# EUROPEAN PATENT APPLICATION

(11) **EP 4 501 313 A1**
(43) Date of publication of application: **05.02.2025**
(21) Application number: 23778099.4
(22) Date of filing: 27.03.2023
(51) Int. Cl.: A61K 9/08, A61K 9/19, A61P 31/00, A61M 5/31, A61M 5/178, A61P 35/00, A61K 39/395, C07K 16/28, C07K 16/46, C12N 15/63, C12N 15/13

(54) **PREPARATIONS CONTAINING ANTI-CLAUDIN18.2/CD3 BISPECIFIC ANTIBODY, PREPARATION METHOD THEREFOR AND USE THEREOF**

(30) Priority: 28.03.2022 CN 202210311376
(71) Applicant: Fortvita Biologics (Singapore) Pte. Ltd., Singapore 189767 (SG)
(72) Inventor: DING, Xuejian, Suzhou, Jiangsu 215123 (CN); MA, Yidong, Suzhou, Jiangsu 215123 (CN)
(74) Representative: Sagittarius IP
(86) International application number: PCT/CN2023/084070
(87) International publication number: WO 2023/185732

(57) **Abstract**

The present invention relates to preparations containing anti-CLDN18.2/CD3 antibody, in particular to pharmaceutical preparations comprising an anti-CLDN18.2/CD3 antibody, a buffer, a stabilizer and a surfactant. In addition, the present invention also relates to the use of the preparations in disease treatment or prevention.

## Description

### TECHNICAL FIELD

The present invention relates to the field of anti-Claudin18.2 and CD3 bispecific antibody formulations. Particularly, the present invention relates to a pharmaceutical formulation, more particularly a stable liquid formulation, a lyophilized formulation, and a reconstituted stable liquid formulation, comprising a bispecific antibody, a method for preparing the pharmaceutical formulation, and therapeutic and/or prophylactic use of the pharmaceutical formulation.

### BACKGROUND

The stability of a drug is one of the key indexes for ensuring its efficacy and safety. A good formulation formula is a key prerequisite to keeping the efficacy and safety of a drug over the shelf life. However, due to the complexity of bispecific antibodies and their degradation pathways, it is currently impossible to predict the formulation conditions required to optimize antibody stability. This is particularly essential considering that different antibodies generally have different structures and CDR sequences, and that such sequence differences will result in different stability properties of the antibodies in a solution. Therefore, based on the stringent requirements for safety and efficacy of antibodies for human use, it is necessary to optimize the formulation for each antibody. Isoform 2 of Claudin-18 (Claudin 18.2 or CLDN18.2) is a highly selective cell lineage marker. Its expression in normal tissues is strictly confined to differentiated epithelial cells of the gastric mucosa and its expression is absent from the gastric stem cell zone. CLDN18.2 is expressed in a considerable portion of primary gastric cancers, and its expression level is retained in gastric metastatic cancer tissue. Expression of CLDN18.2 has also been found in pancreatic cancer in addition to gastric cancer, and it is an ideal target molecule for the treatment of these cancers (Singh, P., Toom, S. & Huang, Y. Anti-CLDN18.2 antibody as new targeted therapy for advanced gastric cancer. J Hematol Oncol 10, 105 (2017). https://doi.org/10.1186/s13045-017-0473-4).

CD3 is a homodimeric or heterodimeric antigen expressed on T cells, which binds to the T cell receptor (TCR) complex and is required for the activation of T cells. Functional CD3 is formed by dimeric association of two of four different chains: ε, ζ, δ, and γ. CD3 dimer arrangements include γ/ε, δ/ε, and ζ/ζ. Antibodies directed against CD3 have been shown to cluster CD3 on T cells, thereby causing the activation of T cells in a manner similar to the engagement of the TCR by peptide-loaded MHC molecules. Thus, anti-CD3 antibodies have been proposed for therapeutic purposes involving the activation of T cells. In addition, it has been proposed that bispecific antibodies capable of binding to CD3 and targeting tumor surface antigens are capable of engaging tumor cells with T cells, thereby directly activating the T cells, releasing granzymes, perforins, and cytokines to kill tumors, and then achieving the therapeutic purpose of inhibiting the tumors. CLDN18.2 is highly expressed in gastric cancer, pancreatic cancer, gastroesophageal junction cancer, and the like, and thus the therapeutic purposes can be achieved by developing bispecific antibodies simultaneously binding to CD3 and Claudin18.2.

Although some bispecific antibody formulations have been proposed, there remains a need in the art for novel pharmaceutical formulations comprising bispecific antibodies that are sufficiently stable and suitable for administration to human subjects. Furthermore, for such bispecific antibody formulations, the simplicity of formulation formulas and ease of use may also be advantageous.

### SUMMARY

The present invention satisfies the need described above by providing a pharmaceutical formulation comprising an anti-Claudin18.2 and CD3 bispecific antibody. The antibody formulation of the present invention exhibits excellent stability against a variety of stability-influencing factors (e.g., temperature and PH).

In one aspect, therefore, the present invention provides a liquid antibody formulation comprising: (i) an anti-Claudin18.2 and CD3 bispecific antibody; (ii) a buffer; (iii) a stabilizer; and (iv) a surfactant. Preferably, the liquid antibody formulation further comprises a chelating agent.

In some aspects, the present invention provides a bispecific antibody, wherein the antibody comprises two binding domains, wherein a first binding domain specifically binds to CLDN18.2, and a second binding domain specifically binds to CD3.

In a preferred embodiment, the first binding domain specifically binding to CLDN18.2 of the present invention is fully human, and/or the second binding domain is humanized.

In some aspects, the bispecific antibody of the present invention is a bispecific antibody with an IgG-like structure.

In one aspect, the present invention relates to the following embodiments.
1. A bispecific antibody, comprising a first antigen-binding domain and a second antigen-binding domain, wherein the first antigen-binding domain specifically binds to CLDN18.2, and the second antigen-binding domain specifically binds to CD3, wherein the first antigen-binding domain comprises three complementarity determining regions A1-HCDR1, A1-HCDR2, and A1-HCDR3 contained in an A1-VH set forth in SEQ ID NO: 4, and three complementarity determining regions A1-LCDR1, A1-LCDR2, and A1-LCDR3 contained in a VL set forth in SEQ ID NO: 9; the second antigen-binding domain comprises three complementarity determining regions A2-HCDR1, A2-HCDR2, and A2-HCDR3 contained in an A2-VH set forth in SEQ ID NO: 30, 22, or 32, and three complementarity determining regions A2-LCDR1, A2-LCDR2, and A2-LCDR3 contained in an A2-VL set forth in SEQ ID NO: 27.
2. The bispecific antibody according to embodiment 1, wherein
   the first antigen-binding domain comprises A1-HCDR1, A1-HCDR2, and A1-HCDR3 set forth in the amino acid sequences of SEQ ID NOs: 1, 2, and 3, respectively, and A1-LCDR1, A1-LCDR2, and A1-LCDR3 set forth in the amino acid sequences of SEQ ID NOs: 6, 7, and 8, respectively; and
   the second antigen-binding domain comprises:
      (i) A2-HCDR1, A2-HCDR2, and A2-HCDR3 set forth in the amino acid sequences of SEQ ID NOs: 19, 20, and 29, respectively, and LCDR1, LCDR2, and LCDR3 set forth in the amino acid sequences of SEQ ID NOs: 24, 25, and 26, respectively; or
      (ii) A2-HCDR1, A2-HCDR2, and A2-HCDR3 set forth in the amino acid sequences of SEQ ID NOs: 19, 20, and 21, respectively, and LCDR1, LCDR2, and LCDR3 set forth in the amino acid sequences of SEQ ID NOs: 24, 25, and 26, respectively; or
      (iii) A2-HCDR1, A2-HCDR2, and A2-HCDR3 set forth in the amino acid sequences of SEQ ID NOs: 19, 31, and 21, respectively, and LCDR1, LCDR2, and LCDR3 set forth in the amino acid sequences of SEQ ID NOs: 24, 25, and 26, respectively.
3. The antibody according to embodiment 1 or 2, wherein the first antigen-binding domain comprises a heavy chain variable region and/or a light chain variable region, wherein the heavy chain variable region
   (i) comprises or consists of an amino acid sequence having at least 90%, 91%, 92%, 93%, 94%, 95%, 96%, 97%, 98%, or 99% identity to the amino acid sequence selected from SEQ ID NO: 4; or
   (ii) comprises or consists of the amino acid sequence selected from SEQ ID NO: 4; or
   (iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 4, wherein preferably, the amino acid changes do not occur in the CDRs; and/or
   the light chain variable region
   (i) comprises or consists of an amino acid sequence having at least 90%, 91%, 92%, 93%, 94%, 95%, 96%, 97%, 98%, or 99% identity to the amino acid sequence selected from SEQ ID NO: 9; or
   (ii) comprises or consists of the amino acid sequence selected from SEQ ID NO: 9; or
   (iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 9, wherein preferably, the amino acid changes do not occur in the CDRs.
4. The antibody according to any one of embodiments 1-3, wherein the second antigen-binding domain comprises a heavy chain variable region and/or a light chain variable region, wherein the heavy chain variable region
   (i) comprises or consists of an amino acid sequence having at least 90%, 91%, 92%, 93%, 94%, 95%, 96%, 97%, 98%, or 99% identity to the amino acid sequence selected from SEQ ID NO: 30, 22, or 32; or
   (ii) comprises or consists of the amino acid sequence selected from SEQ ID NO: 30, 22, or 32; or
   (iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 30, 22, or 32, wherein preferably, the amino acid changes do not occur in the CDRs;
      and/or
   the light chain variable region
   (i) comprises or consists of an amino acid sequence having at least 90%, 91%, 92%, 93%, 94%, 95%, 96%, 97%, 98%, or 99% identity to the amino acid sequence selected from SEQ ID NO: 27; or
   (ii) comprises or consists of the amino acid sequence selected from SEQ ID NO: 27; or
   (iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 27, wherein preferably, the amino acid changes do not occur in the CDRs.
5. The antibody according to any one of embodiments 1-4, further comprising a heavy chain constant region and/or a light chain constant region.
6. The antibody according to any one of embodiments 1-5, wherein the antibody is a bispecific antibody with an IgG-like structure.
7. The antibody according to any one of embodiments 1-6, wherein the heavy chain constant region of the antibody is from IgG1, IgG2, IgG3, or IgG4, preferably IgG1.
8. The antibody according to any one of embodiments 1-6, comprising 2 heavy chain constant regions, wherein one of the heavy chain constant regions A1-HC is linked to the heavy chain variable region A1-VH of the first antigen domain to form a part of a heavy chain binding to CDLN18.2, and the other heavy chain constant region A2-HC is linked to the heavy chain variable region A2-VH of the second antigen-binding domain to form a part of a heavy chain binding to CD3; and 2 light chain constant regions, wherein one of the light chain constant regions A1-LC is linked to the light chain variable region A1-VL of the first antigen domain to form a part of a light chain binding to CLDN18.8, and the other light chain constant region A2-LC is linked to the light chain variable region A2-VL of the second antigen-binding domain to form a part of a light chain binding to CD3.
9. The antibody according to embodiment 8, wherein the A1-HC can be the same as or different from the A2-HC, and/or the A1-LC is the same as or different from the A2-LC.
10. The antibody according to embodiment 8, wherein the A1-VH and A1-VL of the part binding to CLDN18.2 are fully human, and the A2-VH and A2-VL of the part binding to CD3 are humanized.
11. The antibody according to embodiment 8, wherein the part of the heavy chain binding to CLDN18.2
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 37;
   (ii) comprises or consists of the amino acid sequence selected from SEQ ID NO: 37; or
   (iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 37; and/or
   the part of the light chain binding to CLDN18.2
   (i) comprises or consists of an amino acid sequence having at least 85%, 90%, 91%, 92%, 93%, 94%, 95%, 96%, 97%, 98%, or 99% identity to the amino acid sequence selected from SEQ ID NO: 38;
   (ii) comprises or consists of the amino acid sequence selected from SEQ ID NO: 38; or
   (iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 38.
12. The antibody according to embodiment 8 or 11, wherein
   the part of the heavy chain binding to CD3
      (i) comprises or consists of an amino acid sequence having at least 85%, 90%, 91%, 92%, 93%, 94%, 95%, 96%, 97%, 98%, or 99% identity to the amino acid sequence selected from SEQ ID NO: 41, 39, or 42;
      (ii) comprises or consists of the amino acid sequence selected from SEQ ID NO: 41, 39, or 42; or
      (iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 41, 39, or 42; and/or
   the part of the light chain binding to CD3
      (i) comprises or consists of an amino acid sequence having at least 85%, 90%, 91%, 92%, 93%, 94%, 95%, 96%, 97%, 98%, or 99% identity to the amino acid sequence selected from SEQ ID NO: 40;
      (ii) comprises or consists of the amino acid sequence selected from SEQ ID NO: 40; or
      (iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 40.

In some embodiments, the anti-CLDN18.2/CD3 bispecific antibody of the present invention binds to CLDN18.2 (e.g., human CLDN18.2) with high affinity. In some embodiments, the antibody of the present invention specifically binds to CLDN18.2 (e.g., human CLDN18.2), but does not bind to CLDN18.1 (e.g., human CLDN18.1). In some embodiments, the antibody or the antigen-binding fragment thereof of the present invention has higher binding affinity for human CLDN18.2 than that of a known CLDN18.2 antibody, such as zolbetuximab (abbreviated as Zmab) antibody. In some embodiments, the anti-CLDN18.2/CD3 bispecific antibody of the present invention binds to CD3 (e.g., human CD3 or cynomolgus monkey CD3) with desired affinity (e.g., lower). In some embodiments, the antibody of the present invention is capable of binding to both human CD3 and cynomolgus monkey CD3. In some embodiments, the affinity of the antibody is determined by bio-layer interferometry or surface plasmon resonance.

In some embodiments, the anti-CLDN18.2/CD3 bispecific antibody of the present invention is capable of binding to CLDN18.2 on the surface of tumor cells, as well as CD3 on the surface of effector cells. In some embodiments, the binding is determined by flow cytometry.

In one embodiment, the concentration of an anti-CLDN18.2/CD3 protein in the liquid antibody formulation of the present invention is about 0.1-100 mg/mL, preferably about 0.1-10 mg/mL, and more preferably about 0.1-1 mg/mL. In other embodiments, the concentration of the anti-CLDN18.2/CD3 in the liquid antibody formulation of the present invention is about 0.5 mg/mL, 1 mg/mL, 2 mg/mL, 3 mg/mL, 4 mg/mL, or 5 mg/mL.

In some embodiments, the buffer used in the formulation of the present invention is a histidine buffer, particularly a buffer system consisting of histidine and histidine hydrochloride. In some embodiments, the concentration of histidine in the histidine buffer of the present invention is about 0.01-10 mg/mL, particularly about 0.1-1 mg/mL, e.g., about 0.1 mg/mL, 0.2 mg/mL, 0.3 mg/mL, 0.4 mg/mL, 0.5 mg/mL, or 1 mg/mL. In one embodiment, the formulation of the present invention comprises about 0.18 mg/mL histidine. In another embodiment, the histidine buffer used in the formulation of the present invention consists of about 0.18 mg/mL histidine and about 1.84 mg/mL histidine hydrochloride.

In one embodiment, the concentration of the stabilizer in the liquid antibody formulation of the present invention is about 10-100 mg/mL. In one embodiment, the concentration of the stabilizer in the liquid antibody formulation of the present invention is about 20-60 mg/mL, e.g., about 20 mg/mL, 30 mg/mL, 40 mg/mL, 50 mg/mL, or 60 mg/mL.

In one embodiment, the stabilizer is selected from polyols (e.g., sorbitol, mannitol, or a combination thereof), saccharides (e.g., sucrose, trehalose, maltose, or a combination thereof), amino acids (e.g., arginine hydrochloride, methionine, glycine, proline, and a combination thereof or a salt thereof), and any combination thereof. In one embodiment, the stabilizer comprises about 20-80 mg/mL sorbitol, e.g., 20 mg/mL, 25 mg/mL, 30 mg/mL, 35 mg/mL, 40 mg/mL, 45 mg/mL, 50 mg/mL, 55 mg/mL, 60 mg/mL, 70 mg/mL, or 80 mg/mL sorbitol. In another embodiment, the stabilizer comprises about 20-60 mg/mL sucrose, e.g., 20 mg/mL, 30 mg/mL, 40 mg/mL, 50 mg/mL, or 60 mg/mL sucrose. In one preferred embodiment, the stabilizer further comprises arginine, preferably arginine hydrochloride, at a concentration of about 25-200 mM, preferably about 50-150 mM, more preferably about 50-120 mM, and most preferably about 60-100 mM, e.g., about 60 mM, 65 mM, 70 mM, 75 mM, 80 mM, 85 mM, or 90 mM.

In one preferred embodiment, the stabilizer comprises a combination of sorbitol and arginine; or a combination of sucrose and arginine. For example, a combination of about 20-40 mg/mL sorbitol and about 50-100 mM arginine, or a combination of about 30-60 mg/mL sucrose and about 50-100 mM arginine may be used.

In a preferred embodiment, the liquid antibody formulation of the present invention comprises about 30-60 mg/mL (e.g., about 50 mg/mL) sorbitol, or about 20-30 mg/mL (e.g., about 25 mg/mL) sorbitol, and 80-90 mM (e.g., about 85 mM) arginine.

In one embodiment, the concentration of the surfactant in the liquid antibody formulation of the present invention is about 0.1-1 mg/mL. In one embodiment, the concentration of the surfactant in the liquid antibody formulation of the present invention is about 0.2-0.8 mg/mL, e.g., about 0.2 mg/mL, 0.3 mg/mL, 0.4 mg/mL, 0.5 mg/mL, 0.6 mg/mL, 0.7 mg/mL, or 0.8 mg/mL.

In one embodiment, the surfactant is a non-ionic surfactant. In one embodiment, the surfactant is selected from polysorbate surfactants, poloxamer, and polyethylene glycol. In one embodiment, the surfactant is selected from polysorbate surfactants. In one specific embodiment, the surfactant in the liquid antibody formulation of the present invention is polysorbate-80.

### Concentration and type of chelating agent:

In one embodiment, the concentration of the chelating agent in the liquid antibody formulation of the present invention is about 0.005-0.05 mg/mL. In one embodiment, the concentration of the chelating agent in the liquid antibody formulation of the present invention is about 0.008-0.018 mg/mL, e.g., about 0.008 mg/mL, 0.009 mg/mL, 0.010 mg/mL, 0.012 mg/mL, 0.014 mg/mL, or 0.018 mg/mL.

In one embodiment, the chelating agent is a carboxylic acid chelating agent. In one embodiment, the chelating agent is selected from edetate disodium, nitrilotriacetic acid, diethylenetriaminepentaacetic acid, citric acid, tartaric acid, gluconic acid, hydroxyethylethylenediaminetriacetic acid, and dihydroxyethylglycine. In one embodiment, the chelating agent is selected from edetate disodium.

In one embodiment, the liquid formulation has a pH value of about 5.0-6.0. In some embodiments, the liquid formulation has a pH value of any value of about 5.0-6.0, e.g., about 5.0, 5.2, 5.4, 5.6, 5.8, or 6.0. Preferably, the formulation has a pH of 5.2±0.2 or 5.5±0.2, preferably a pH of 5.2.

In one embodiment, the liquid antibody formulation of the present invention comprises:
(i) about 0.1-100 mg/mL anti-CLDN18.2/CD3 protein;
(ii) about 0.1-2 mg/mL histidine;
(iii) about 30-70 mg/mL sorbitol;
(iv) about 0.1-1 mg/mL polysorbate 80; and
(v) optionally, about 0.008-0.018 mg/mL edetate disodium,
   wherein the liquid formulation has a pH of about 5.0-5.5, e.g., about 5.2;
   for example, the liquid antibody formulation comprises:
      (i) about 0.1-10 mg/mL, e.g., 0.1-1 mg/mL, anti-CLDN18.2/CD3 protein;
      (ii) about 1.55 mg/mL histidine;
      (iii) about 40-60 mg/mL sorbitol or sucrose, preferably 50 mg/mL sorbitol or 30-50 mg/mL sucrose;
      (iv) about 0.2-0.8 mg/mL, e.g., 0.2-0.6 mg/mL, polysorbate 80; and
      (v) optionally, about 0.008-0.018 mg/mL, e.g., 0.01 mg/mL, edetate disodium,
   wherein the liquid formulation has a pH of about 5.0-5.5, e.g., about 5.2;
   alternatively, the liquid antibody formulation comprises
      (i) about 1 mg/mL anti-CLDN18.2/CD3 protein, about 1.55 mg/mL histidine, about 50.00 mg/mL sorbitol, and about 0.2 mg/mL polysorbate 80, with a pH of about 5.2; or
      (ii) about 50 mg/mL anti-CLDN18.2/CD3 protein, about 1.55 mg/mL histidine, about 50.00 mg/mL sorbitol, about 0.01 mg/mL edetate disodium, and about 0.2 mg/mL polysorbate 80, with a pH of about 5.2; or
      (iii) about 50 mg/mL anti-CLDN18.2/CD3 protein, about 0.82 mg/mL sodium acetate, about 50.00 mg/mL sorbitol, and about 0.2 mg/mL polysorbate 80, with a pH of 5.2.

The liquid formulation of the present invention may be stably stored for a long period of time, e.g., at least 24 months or longer. In one embodiment, the liquid formulation of the present invention may be stably stored at about -80 °C to about 45 °C, e.g., about -80 °C, about -30 °C, about -20 °C, about 0 °C, about 5 °C, about 25 °C, about 35 °C, about 38 °C, about 40 °C, about 42 °C, or about 45 °C for at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer.

In one embodiment, the liquid formulation of the present invention may be stably stored for at least 24 months. In yet another embodiment, the liquid formulation of the present invention is stable at a temperature of at least 40 °C. In yet another embodiment, the liquid formulation of the present invention remains stable at about 2 °C to 8 °C for at least 3 months, preferably at least 12 months, and more preferably at least 24 months. In one embodiment, the liquid formulation of the present invention remains stable at room temperature or about 25 °C for at least 2 months, preferably at least 3 months, and more preferably at least 6 months. In yet another embodiment, the liquid formulation of the present invention remains stable at about 40 °C for at least 2 weeks, preferably at least 1 month. In one embodiment, the stability of the formulation may be indicated by detecting changes in the appearance, visible particles, protein content, turbidity, purity, and/or charge variants of the formulation. In one embodiment, the stability of the liquid formulation of the present invention may be detected in a forced high temperature stress test, e.g., after storage at 40±2 °C for at least 1 week, 2 weeks, or preferably 1 month, or in an accelerated test, e.g., after storage at 25±2 °C for at least 1 month or 2 months, or in a long-term test, e.g., after storage at 5±3 °C for at least 2 months or 3 months, or in a shaking test (e.g., shaking at room temperature in the dark at 650 r/min for 5 days), and/or in a freezing-thawing test (e.g., 6 freezing-thawing repeats at -30 °C/room temperature). In one embodiment, the stability of the liquid formulation of the present invention is detected relative to an initial value, for example, an initial value on day 0 of storage, or an initial value prior to a shaking or freezing-thawing test.

In one embodiment, the stability of the liquid formulation of the present invention is visually inspected after storage, after a shaking test, or after a freezing-thawing test, wherein the liquid formulation of the present invention remains a clear to slightly opalescent, colorless to yellowish liquid free of particles in appearance. In one embodiment, no visible particles exist in the formulation upon visual inspection under a clarity detector. In one embodiment, the stability of the liquid formulation of the present invention is tested after storage, after a shaking test, or after a freezing-thawing test by determining the change in protein content, wherein the change rate in protein content is not more than 20%, preferably not more than 10%, e.g., 7%-8%, and more preferably not more than 5%, 2%, or 1%, relative to an initial value, as measured, for example, by ultraviolet spectrophotometry (UV). In one embodiment, the stability of the liquid formulation of the present invention is tested after storage, after a shaking test, or after a freezing-thawing test by determining the change in turbidity of the liquid formulation of the present invention, wherein the change is not more than 0.06, preferably not more than 0.05, and more preferably not more than 0.04 or not more than 0.02, relative to an initial value, as measured, for example, by the OD_{350 mm} method. In one embodiment, the stability of the liquid formulation of the present invention is tested after storage, after a shaking test, or after a freezing-thawing test by determining the change in purity of the liquid formulation of the present invention, wherein the change in monomer purity (or change in main peak) is not more than 10%, e.g., not more than 5%, 4%, or 3%, e.g., not more than 2%, preferably not more than 1%, relative to an initial value, as measured by size exclusion chromatography-high performance liquid chromatography (SEC-HPLC). In one embodiment, the stability of the liquid formulation of the present invention is tested after storage, after a shaking test, or after a freezing-thawing test by determining the change in purity of the liquid formulation of the present invention, wherein the change in monomer purity (or change in main peak) is reduced by no more than 10%, e.g., no more than 5%, 4%, 3%, 2%, or 1%, relative to an initial value, as measured by non-reduced capillary electrophoresis-sodium dodecyl sulfate (CE-SDS). In one embodiment, the stability of the liquid formulation of the present invention is tested after storage, after a shaking test, or after a freezing-thawing test by cation exchange high performance liquid chromatography (CEX-HPLC), wherein the sum of changes in charge variants (main species, acidic species, and basic species) of the antibody is not more than 50%, e.g., not more than 40%, 30%, 20%, 10%, or 5%, and/or the change in main species is not more than 20%, 15%, 10%, 8%, or 5%, relative to an initial value. In one embodiment, the stability of the liquid formulation of the present invention is tested after storage, after a shaking test, or after a freezing-thawing test by direct ELISA, wherein the relative binding activity of the antibody is 70%-130%, e.g., 70%, 80%, 90%, 93%, 95%, 98%, 100%, 103%, 105%, 108%, 110%, 115%, 120%, 125%, or 130%, preferably 90%-110%, relative to an initial value.

In one embodiment, the liquid formulation of the present invention is stable after storage, e.g., at 25 °C for at least 2 months or at 40±2 °C for 1 month, and preferably has one or more of the following characteristics relative to an initial value on day 0 of storage:
(i) a main peak change less than 1%, and/or a purity greater than 96%, preferably greater than 97% or 98% in the formulation as measured by SEC-HPLC;
(ii) a main peak change less than 2%, and/or a purity greater than 96%, preferably greater than 97% or 98% in the formulation as measured by non-reduced CE-SDS;
(iii) a sum of changes in species (main species, acidic species, and basic species) not more than 40% and/or a change in the main species not more than 20% of the anti-CLDN18.2/CD3 protein in the formulation as measured by CEX-HPLC,
   for example, a sum of changes not more than about 40% (e.g., not more than 35%, 30%, 25%, 20%, 15%, or 10%) or a change in the main species not more than 20% (e.g., not more than 15%, 12%, 10%, or 8%) after storage at 40±2 °C for 1 month, or
   for example, a sum of changes not more than about 20% (e.g., not more than 15%, 14%, 13%, or 12%) or a change in the main species not more than about 15% (e.g., not more than 10%, 8%, 7%, 6%, or 5%) after storage at 25 °C for 2 months; and
(iv) a relative binding activity of the anti-CLDN18.2/CD3 protein in the formulation of 70%-130%, e.g., 90%, 93%, 95%, 98%, 100%, 103%, 105%, 108%, 110%, 115%, or 120%, such as 90%-110%, as measured by ELISA. In a preferred embodiment, the liquid formulation of the present invention is stable at shaking and/or repeated freezing-thawing.

Preferably, the formulation is stable at shaking or repeated freezing-thawing, e.g., after shaking at room temperature in the dark at 650 r/min for 5 days or after 6 freezing-thawing repeats at -30 °C/room temperature, and has one or more of the following characteristics:
(i) a main peak change less than 1%, and/or a purity greater than 96%, preferably greater than 97%, 98%, or 99% in the formulation as measured by SEC-HPLC;
(ii) a main peak change less than 2%, and/or a purity greater than 96%, preferably greater than 97% or 98% in the formulation as measured by non-reduced CE-SDS;
(iii) a sum of changes in species (main species, acidic species, and basic species) not more than 2% of the anti-CLDN18.2/CD3 protein in the formulation as measured by CEX-HPLC; and
(iv) a relative binding activity of the anti-CLDN18.2/CD3 protein in the formulation of 70%-130%, e.g., 90%-110%, as measured by ELISA.

In one aspect, the liquid formulation of the present invention is a pharmaceutical formulation, preferably an injection, and more preferably a subcutaneous injection or an intravenous injection. In one embodiment, the liquid formulation is an intravenous infusion.

In another aspect, the present invention provides a solid antibody formulation obtained by solidifying the liquid antibody formulation of the present invention. The solidification treatment is implemented by, e.g., crystallization, spray drying, or lyophilization. In one preferred embodiment, the solid antibody formulation is, e.g., in the form of a lyophilized powder for injection. The solid antibody formulation may be reconstituted in a suitable vehicle prior to use to give a reconstituted formulation of the present invention. The reconstituted formulation is also a liquid antibody formulation of the present invention. In one embodiment, the suitable vehicle is selected from water for injection, organic solvents for injection (including but not limited to, oil for injection, ethanol, propylene glycol, and the like), or combinations thereof.

In one aspect, the present invention provides a delivery device comprising the liquid antibody formulation or the solid antibody formulation of the present invention. In one embodiment, the delivery device of the present invention is provided in the form of a pre-filled syringe comprising the liquid antibody formulation or the solid antibody formulation of the present invention, e.g., for use in intravenous, subcutaneous, intradermal or intramuscular injection, or intravenous infusion.

In another aspect, the present invention provides a method for delivering the anti-CLDN18.2/CD3 protein to a subject, e.g., a mammal, comprising administering the liquid antibody formulation or the solid antibody formulation of the present invention to the subject, the delivery being implemented, e.g., using a delivery device in the form of a pre-filled syringe.

In another aspect, the present invention provides a method for preventing or treating a tumor (e.g., cancer) in a subject using the liquid antibody formulation or the solid antibody formulation of the present invention, comprising administering to the subject an effective amount of the anti-CLDN18.2×CD3 bispecific antibody or the fragment thereof (preferably the antigen-binding fragment), a pharmaceutical composition, a pharmaceutical combination, or a kit of the present invention.

The present invention further provides a method for administering to a subject the liquid antibody formulation or the solid antibody formulation of the present invention or the delivery device (e.g., the pre-filled syringe) or a medicament comprising the liquid antibody formulation or the solid antibody formulation.

Other embodiments of the present invention will become apparent by reference to the detailed description hereinafter.

### BRIEF DESCRIPTION OF THE DRAWINGS

The preferred embodiments of the present invention described in detail below will be better understood when read in conjunction with the following drawings. For the purpose of illustrating the present invention, currently, preferred embodiments are shown in the drawings. However, it should be understood that the present invention is not limited to the precise arrangements and means of the embodiments shown in the drawings.
FIG. 1 shows a schematic structural diagram of the bispecific antibody used in the examples.
FIG. 2 shows that the bispecific antibody of the present invention specifically kills CLDN18.2-positive gastric cancer cells NUGC-4.
FIG. 3 shows that the bispecific antibody specifically kills CLDN18.2-positive pancreatic cancer cells DAN-GCLDN18.2.
FIG. 4 shows that the bispecific antibody has no non-specific killing against cells negative for CLDN18.2 expression.
FIG. 5 shows the *in vivo* efficacy results of the bispecific antibody in an NUGC-4 gastric cancer humanization model.
FIG. 6 shows the *in vivo* efficacy results of the bispecific antibody in a DAN-G-CLDN18.2 pancreatic cancer humanization model.
FIG. 7 shows the PK of the bispecific antibody in mice.
FIG. 8 shows the effect of pH value changes in the formula: (A) the trend of change in protein content (UV method); (B) the trend of change in purity (SEC-HPLC method); (C) the trend of change in purity (nrCE-SDS method); (D) the trend of change in charge heterogeneity-acidic species (iCIEF method); (E) the trend of change in charge heterogeneity-main species (iCIEF method); and (F) the trend of change in charge heterogeneity-basic species (iCIEF method).
FIG. 9 shows the effect of different formula compositions: (A) the trend of change in protein content (UV method); (B) the trend of change in purity (SEC-HPLC method); (C) the trend of change in purity (nrCE-SDS method); (D) the trend of change in charge heterogeneity-acidic species (iCIEF method); (E) the trend of change in charge heterogeneity-main species (iCIEF method); and (F) the trend of change in polysorbate 80 content (HPLC-FLD method).

### DETAILED DESCRIPTION

### Definitions

Unless otherwise defined, all technical and scientific terms used herein have the same meaning as those commonly understood by those of ordinary skill in the art. For the purposes of the present invention, the following terms are defined below.

The term "about" used in combination with a numerical value is intended to encompass the numerical values in a range from a lower limit less than the specified numerical value by 5% to an upper limit greater than the specified numerical value by 5%.

The term "and/or", when used to connect two or more options, should be understood to refer to any one of the options or any two or more of the options.

As used herein, the term "comprise" or "include" is intended to mean that the elements, integers, or steps are included, but not to the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

The term "CLAUDIN" or "CLDN" used herein is the most important skeleton protein that determines the structures of tight junctions between cells, and it is involved in adhesion junctions and plays an important role in the metastasis and invasion of tumor cells. Claudin proteins are widely present in mammalian epithelial and endothelial cells, primarily on the lateral surface of epithelial cells and on the plasma membrane of basal cells. Different Claudin proteins have their respective specific expression in different tissues, wherein the Claudin18 (CLDN18) gene is located at 3q22.3, has a molecular weight of 24 kDa, comprises 261 amino acid residues, and is a member of the Claudins superfamily, and its protein structure comprises 2 extracellular loops and 4 transmembrane domains. Two subtypes of human CLDN18 or Claudin18 protein are Claudin18.1 or CLDN18.1 (UniProt ID: P56856-1) and Claudin18.2 or CLDN18.2 (UniProt ID: P56856-2). In the primary structural sequences of the two proteins, only the amino acid residues at some positions from the N-terminus signal peptide to the extracellular loop 1 (Loop1) structure are different, and especially at the extracellular loop 1, CLDN18.1 and CLDN18.2 differ by only 8 amino acids. The intergeneric sequence homology of the two subtypes of CLDN18 protein is also very high. The sequences of the extracellular loop 1 of CLDN18.2 are completely identical in different species such as humans, mice, and rhesus monkeys, and the homology of human and mouse CLDN18.2 proteins reaches 84%, indicating that the sequence of CLDN18.2 protein is extremely conservative (O. Tureci. et al., Gene, 481:83-92, 2011). CLDN18.2 or any variants and isoforms thereof may be isolated from cells or tissues in which they are naturally expressed or recombinantly produced using techniques well known in the art and/or those described herein. In one embodiment, the CLDN18.2 described herein is human CLDN18.2.

The term "anti-CLDN18.2 antibody", "anti-CLDN18.2", "CLDN18.2 antibody", or "antibody binding to CLDN18.2" used herein refers to an antibody capable of binding to (human) CLDN18.2 with sufficient affinity such that the antibody can be used as a therapeutic agent targeting (human) CLDN18.2. In one embodiment, the (human) CLDN18.2 antibody binds to (human) CLDN18.2 with high affinity *in vitro* or *in vivo.* In one embodiment, the (human) CLDN18.2 antibody does not bind to CLDN18.1. In one embodiment, the (human) CLDN18.2 antibody binds to cells expressing CLDN18.2 but does not bind to cells expressing CLDN18.1. In some embodiments, the binding is determined, e.g., by radioimmunoassay (RIA), bio-layer interferometry (BLI), MSD assay, surface plasmon resonance (SPR), or flow cytometry.

The term "CD3" as used herein refers to an antigen expressed on a T cell as part of a multi-molecular T cell receptor (TCR), and it consists of a homodimer or heterodimer formed from two of the following four receptor chains: CD3-ε, CD3-δ, CD3-ζ, and CD3-γ. Human CD3-εn (hCD3ε) comprises an amino acid sequence described in UniProtKB/Swiss-Prot: P07766.2. Human CD3-δ (hCD3δ) comprises an amino acid sequence described in UniProtKB/Swiss-Prot: P04234.1. In some embodiments, the CD3 described herein refers to CD3 from a human or cynomolgus monkey.

The term "antibody binding to CD3" or "anti-CD3 antibody" as used herein includes an antibody and an antigen-binding fragment thereof that specifically recognize or bind to a single CD3 subunit (e.g., ε, δ, γ, or ζ), as well as an antibody and an antigen-binding fragment thereof that specifically recognize and bind to a dimeric complex of two CD3 subunits (e.g., γ/ε, δ/ε, and ζ/ζ CD3 dimers). The antibody and the antigen-binding fragment of the present invention may bind to soluble CD3, binding CD3, and/or CD3 expressed on the cell surface. The soluble CD3 includes native CD3 proteins and recombinant CD3 protein variants, e.g., monomeric and dimeric CD3 structures that lack a transmembrane region or otherwise do not bind to the cell membrane. The present invention provides an antibody binding to human and cynomolgus monkey CD3 with lower or undetectable binding affinity, thereby capable of activating human and cynomolgus monkey T cells. In some embodiments, the binding is determined, e.g., by radioimmunoassay (RIA), bio-layer interferometry (BLI), MSD assay, surface plasmon resonance (SPR), or flow cytometry.

The term "CD3 expressed on the cell surface" refers to one or more CD3 proteins that are expressed on the cell surface *in vivo* or *in vitro,* such that at least part of the CD3 protein is exposed on the extracellular side of the cell membrane and is accessible to an antigen-binding portion of an antibody. "CD3 expressed on the cell surface" incudes CD3 proteins contained in the environment of a functional T cell receptor in the cell membrane. The term "CD3 expressed on the cell surface" includes CD3 proteins expressed as a part of a homodimer or heterodimer (e.g., δ/ε, γ/ε, and ζ/ζ CD3 dimers) on the cell surface.

Effector cells include effector T cells (T lymphocytes), such as CD4+ T cells, CD8+ T cells, Th1, Th2, and regulatory T cells (Tregs). The effector cells may also include natural killer cells, macrophages, granulocytes, plasma cells, or B cells (lymphocytes).

As used herein, the term "antibody" is used in the broadest sense, and refers to a protein comprising an antigen-binding site and encompasses natural and artificial antibodies with various structures, including but not limited to intact antibodies and antigen-binding fragments of antibodies.

The "bispecific" antibody refers to an antibody having two antigen-binding sites, each of which binds to a different epitope of the same antigen or to a different epitope of a different antigen. In one embodiment, the bispecific antibody has binding specificities for a first antigen and a second antigen.

The bispecific antibody formats include IgG-like and non-IgG-like antibodies (Fan et al., (2015) Journal of Hematology & Oncology. 8: 130). The most common type of IgG-like antibodies comprises two Fab regions and one Fc region, and the heavy and light chains of each Fab may be from a separate monoclonal antibody. Non-IgG-like bispecific antibodies lack Fc regions, and each antigen or target-binding domain thereof may be a Fab, or a single-chain variable fragment (scFv), or a fusion protein that mimics variable domains of two antibodies. The different binding domains are linked together by a peptide linker, chemical coupling, non-covalent linkage, or other methods. These formats comprise bispecific T-cell engagers (BiTEs).

The bispecific antibody of the present invention may be prepared using any bispecific antibody formats or techniques. For example, an antibody or a fragment thereof having binding specificity for a first antigen may be functionally linked to one or more other molecular entities, such as another antibody or an antibody fragment having binding specificity for a second antigen, (e.g., by chemical coupling, genetic fusion, non-covalent association, or other methods), to produce a bispecific antibody. Specific exemplary bispecific formats that may be used in the context of the present invention include, but are not limited to, the following: scFv-based or diabody bispecific formats, IgG-scFv fusion, dual-variable domain (DVD)-Ig, quadroma, knobs-into-holes, ordinary light chains (e.g., ordinary light chains with knobs-into-holes, etc.), CrossMab, CrossFab, (SEED)body, Duobody, IgG1/IgG2, dual action Fab (DAF)-IgG, and Mab² bispecific formats.

The term "linker" as used herein refers to any molecule that enables a direct linkage of different portions of a bispecific antibody. Examples of linkers to establish covalent linkages between different antibody portions include peptide linkers and non-proteinaceous polymers including, but not limited to, polyethylene glycol (PEG), polypropylene glycol, polyalkylene oxide and copolymers of polyethylene glycol and polypropylene glycol.

The term "peptide linker" according to the present invention refers to an amino acid sequence that links the amino acid sequence of a first portion of an antibody to a second portion of the antibody. For example, the peptide linker may link a first (variable and/or binding) domain of to a second (variable and/or binding) domain of the antibody. For example, the peptide linker may also link one portion of the antibody to another portion of the antibody, for example, an antigen-binding domain to an Fc domain or a fragment thereof. Preferably, the peptide linker has a length sufficient to link two entities in a manner that maintains their conformation relative to each other without interference with the desired activities.

The peptide linker may or may not predominantly comprise the following amino acid residues: Gly, Ser, Ala or Thr. Useful linkers include glycine-serine polymers including, for example, (GS)n, (GSGGS)n, (GGGGS)n, (GGGS)n, and (GGGGS)nG, wherein n is an integer of at least 1 (and preferably 2, 3, 4, 5, 6, 7, 8, 9, or 10). Useful linkers also include glycine-alanine polymers, alanine-serine polymers, and other flexible linkers.

The term "valent" according to the present invention refers to the specified number of binding sites present in an antibody molecule. Thus, the terms divalent, trivalent and tetravalent indicate the presence of two, three and four binding sites, respectively, in an antibody construct. The bispecific antibodies according to the present invention are at least bivalent and may be multivalent, e.g., bivalent, trivalent, tetravalent, or hexavalent.

The term "binding domain" as used herein refers to any portion of a bispecific antibody that binds to a particular target or antigen. The binding domain is an antigen-binding site. The binding domain may be, for example, an antibody or immunoglobulin per se or an antibody fragment. Such binding domains may or may not have a tertiary structure independent of the remainder of BsAB, and may or may not bind to their targets as separate entities.

In certain exemplary embodiments of the present invention, each antigen-binding domain of the bispecific antibody comprises a heavy chain variable region VH and a light chain variable region VL. In a bispecific antibody comprising a first antigen-binding domain and a second antigen-binding domain, the VH, VL, or CDRs of the first antigen-binding domain may be represented by the prefix "A1", and the VH, VL, or CDRs of the second antigen-binding domain may be represented by the prefix "A2". For example, the heavy chain CDRs (HCDRs) of the first antigen-binding domain may be referred to herein as A1-HCDR1, A1-HCDR2, and A1-HCDR3, and the heavy chain CDRs of the second antigen-binding domain may be referred to herein as A2-HCDR1, A2-HCDR2, and A2-HCDR3. Similarly, the heavy chain variable region VH of the first antigen-binding domain is referred to herein as A1-VH, and the heavy chain variable region VH of the second antigen-binding domain is referred to herein as A2-VH. The light chain CDRs (HCDRs) of the first antigen-binding domain may be referred to herein as A1-LCDR1, A1-LCDR2, and A1-LCDR3, and the light chain CDRs of the second antigen-binding domain may be referred to herein as A2-LCDR1, A2-LCDR2, and A2-LCDR3. Similarly, the light chain variable region VL of the first antigen-binding domain is referred to herein as A1-VL, and the light chain variable region VL of the second antigen-binding domain is referred to herein as A2-VL.

The term "antibody fragment" includes a portion of an intact antibody. In a preferred embodiment, the antibody fragment is an antigen-binding fragment.

"Antigen-binding fragment" refers to a molecule different from an intact antibody, which comprises a portion of the intact antibody and binds to an antigen to which the intact antibody binds. Examples of the antibody fragments include, but are not limited to, an Fv, a Fab, a Fab', a Fab'-SH, a F(ab')₂, a dAb (domain antibody), a linear antibody, a single-chain antibody (e.g., scFv), a single-domain antibody (e.g., VHH), a bi-valent antibody or a fragment thereof, or a camelid antibody.

The term "antigen" refers to a molecule that induces an immune response. Such an immune response may involve antibody production or activation of specific immune cells, or both. Those skilled will understand that any macromolecules, including substantially all proteins or peptides, may be used as antigens. In addition, an antigen may be derived from recombinant or genomic DNA. As used herein, the term "epitope" refers to a moiety of an antigen (e.g., CLDN18.2) that specifically interacts with an antibody molecule.

"Antibody that binds to the same or overlapping epitope" as a reference antibody refers to an antibody that blocks 50%, 60%, 70%, 80%, 90%, or 95% or more of the binding of the reference antibody to its antigen in a competition assay. Conversely, the reference antibody blocks 50%, 60%, 70%, 80%, 90%, or 95% or more of the binding of the antibody to its antigen in a competition assay.

An antibody that competes with a reference antibody for binding to its antigen refers to an antibody that blocks 50%, 60%, 70%, 80%, 90%, or 95% or more of the binding of the reference antibody to its antigen in a competition assay. Conversely, the reference antibody blocks 50%, 60%, 70%, 80%, 90%, or 95% or more of the binding of the antibody to its antigen in a competition assay. Numerous types of competitive binding assays may be used to determine whether an antibody competes with another antibody, such as direct or indirect solid-phase radioimmunoassay (RIA), direct or indirect solid-phase enzyme immunoassay (EIA), and sandwich competition assay.

An antibody that inhibits (e.g., competitively inhibits) the binding of a reference antibody to its antigen refers to an antibody that inhibits 50%, 60%, 70%, 80%, 90%, or 95% or more of the binding of the reference antibody to its antigen. Conversely, the reference antibody inhibits 50%, 60%, 70%, 80%, 90%, or 95% or more of the binding of the antibody to its antigen. The binding of an antibody to its antigen may be measured by affinity (e.g., equilibrium dissociation constant). Methods for determining affinity are known in the art.

An antibody that shows identical or similar binding affinity and/or specificity as a reference antibody refers to an antibody that is capable of having at least 50%, 60%, 70%, 80%, 90%, or 95% or more of the binding affinity and/or specificity of the reference antibody. This can be determined by any method known in the art for determining binding affinity and/or specificity.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). CDRs are primarily responsible for binding to antigen epitopes. CDRs of heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR may be determined using any one or a combination of many well-known antibody CDR assignment systems including, e.g., Chothia based on the three-dimensional structure of antibodies and the topology of the CDR loops (Chothia et al., (1989) Nature, 342: 877-883; Al-Lazikani et al., "Standard conformations for the canonical structures of immunoglobulins"", Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

For example, according to different CDR determination schemes, the residues of each CDR are described as follows.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

CDRs can also be determined based on having the same Kabat numbering positions as a reference CDR sequence (e.g., any one of the exemplary CDRs of the present invention).

Unless otherwise stated, the term "CDR" or "CDR sequence" used herein encompasses CDR sequences determined by any one of the schemes described above.

Unless otherwise stated, residue positions of an antibody variable region (including heavy chain variable region residues and light chain variable region residues) used herein are numbered according to the Kabat numbering system (Kabat et al., Sequences of Proteins of Immunological Interest, 5th Ed., Public Health Service, National Institutes of Health, Bethesda, Md. (1991)).

In one embodiment, the heavy chain variable region CDRs of the antibody of the present invention are determined according to the following rules:
The VH CDR1 is determined according to AbM rules, and VH CDR2 and VH CDR3 are both determined according to Kabat rules.

In one embodiment, the light chain variable region CDRs of the antibody of the present invention are determined according to Kabat rules.

In one embodiment, the heavy chain variable region CDRs of the antibody of the present invention are determined according to the following rules: the VH CDR1 is determined according to AbM rules, and the VH CDR2 and CDR3 are both determined according to Kabat rules; and the light chain variable region CDRs are determined according to Kabat rules.

It should be noted that boundaries of CDRs in variable regions of the same antibody determined by different assignment systems may differ. That is, CDR sequences of variable regions of the same antibody defined by different assignment systems differ. Therefore, when it comes to defining an antibody with specific CDR sequences defined in the present invention, the scope of antibody also encompasses such antibodies whose variable region sequences comprise the specific CDR sequences but having claimed CDR boundaries different from the specific CDR boundaries defined by the present invention due to a different protocol (e.g., different assignment system rules or their combinations) applied.

Antibodies with different specificities (i.e., different binding sites for different antigens) have different CDRs (under the same assignment system). However, although CDRs vary from antibody to antibody, only a limited number of amino acid positions within the CDRs are directly involved in antigen binding. The smallest overlapping region can be determined using at least two of the Kabat, Chothia, AbM, Contact, and North methods, thereby providing a "minimal binding unit" for antigen binding. The minimal binding unit may be a sub-portion of the CDR. As will be clear to those skilled in the art, residues in the remaining portions of the CDR sequences can be determined by the structure and protein folding of the antibody. Therefore, variants of any CDR presented herein are also considered. For example, in a variant of one CDR, the amino acid residue of the minimal binding unit may remain unchanged, while the remaining CDR residues defined according to Kabat or Chothia may be replaced by conservative amino acid residues.

The term "Fc region" is used herein to define the constant regions of CH2 and CH3 of immunoglobulin heavy chains, and includes Fc regions of native sequences and variant Fc regions. The native Fc region can bind to different Fc receptors on the immune cell surface, thereby causing CDC\ADCC\ADCP effector functions. Such effector functions generally require that the Fc region is associated with a binding domain (e.g., an antibody variable domain). In some embodiments, the Fc region is mutated to enhance its CDC\ADCC\ADCP effector functions. In some embodiments, the Fc region is mutated to attenuate or delete its CDC\ADCC\ADCP effector functions.

"Antibody in the form of IgG" refers to the IgG form that the heavy chain constant region of the antibody belongs to. Heavy chain constant regions of all antibodies of the same type are identical, and heavy chain constant regions of antibodies of different types are different. For example, an antibody in the form of IgG4 means that the heavy chain constant region of the antibody is from IgG4, or an antibody in the form of IgG1 means that the heavy chain constant region of the antibody is from IgG1.

"Humanized" antibody refers to an antibody comprising amino acid residues from non-human CDRs and amino acid residues from human FRs. In some embodiments, a humanized antibody will comprise at least one, or generally two of substantially all variable domains in which all or substantially all CDRs (e.g., CDRs) correspond to those of a non-human antibody, and all or substantially all FRs correspond to those of a human antibody. A humanized antibody may optionally comprise at least a portion of an antibody constant region derived from a human antibody. The "humanized form" of an antibody (such as a non-human antibody) refers to an antibody that has been humanized.

"Human antibody", "fully human antibody" and "fully humanized antibody" are used interchangeably, and refer to an antibody having an amino acid sequence that corresponds to the amino acid sequence of an antibody generated by a human or human cell or derived from a non-human source that utilizes human antibody libraries or other human antibody encoding sequences. This definition of a human antibody explicitly excludes humanized antibodies comprising non-human antigen-binding residues.

As used herein, the term "bind to" or "specifically bind to" means that the binding effect is selective for antigens and may be distinguished from unwanted or non-specific interactions. The ability of an antigen-binding site to bind to a particular antigen may be determined by enzyme-linked immunosorbent assay (ELISA) or conventional binding assays known in the art, such as radioimmunoassay (RIA), bio-layer interferometry, MSD assay, or surface plasmon resonance (SPR).

The term "therapeutic agent" described herein encompasses any substance that is effective in preventing or treating a tumor, e.g., cancer, including a chemotherapeutic agent, a cytokine, an angiogenesis inhibitor, a cytotoxic agent, other antibodies, a small molecule drug, or an immunomodulatory agent (e.g., an immunosuppressant).

The term "cytotoxic agent" used herein refers to a substance that inhibits or prevents cell functions and/or causes cell death or cell destruction.

"Chemotherapeutic agents" include chemical compounds useful in the treatment of cancer or immune system diseases.

The term "small molecule drug" refers to an organic compound having a low molecular weight and capable of regulating biological processes. "Small molecule" is defined as a molecule with a molecular weight of less than 10 kD, usually less than 2 kD, and preferably less than 1 kD. The small molecule includes, but is not limited to, inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation, and are less likely to induce an immune response compared with large molecules.

The term "immunomodulatory agent" used herein refers to a natural or synthetic active agent or drug that suppresses or modulates an immune response. The immune response may be a humoral response or a cellular response. The immunomodulatory agent includes an immunosuppressant. In some embodiments, the immunomodulatory agent of the present invention includes an immune checkpoint inhibitor or an immune checkpoint agonist.

The term "effective amount" refers to an amount or dosage of the antibody, fragment, composition, or combination of the present invention which generates expected effects in a patient in need of treatment or prevention after being administered to the patient in a single dose or multiple doses.

"Therapeutically effective amount" refers to an amount effective to achieve a desired therapeutic result at a necessary dose for a necessary period of time. The therapeutically effective amount is also such an amount that any toxic or undesired effect of the antibody, fragment thereof, composition, or combination is inferior to the therapeutically beneficial effect. The "therapeutically effective amount" preferably inhibits a measurable parameter (e.g., tumor volume) by at least about 40%, and even more preferably by at least about 50%, 55%, 60%, 65%, 70%, 75%, 80%, 85%, 90%, or even 100%, relative to untreated subjects.

"Prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate the percent identity between the sequences. In one preferred embodiment, the percent identity between two amino acid sequences is determined with the Needlema and Wunsch algorithm ((1970) J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5. The percent identity between two amino acid sequences or nucleotide sequences can also be determined with a PAM120 weighted remainder table, a gap length penalty of 12, and a gap penalty of 4, using the E. Meyers and W Miller algorithm ((1989) CABIOS, 4: 11-17) that has been incorporated into the ALIGN program (version 2.0). Additionally or alternatively, the nucleic acid sequences and protein sequences described herein can be further used as "query sequences" to perform searches against public databases to, e.g., identify other family member sequences or related sequences.

As used herein, the term "hybridization under stringency conditions (such as low stringency, medium stringency, high stringency or extreme stringency)" describes hybridization and washing conditions. Instructions for performing hybridization reactions may be found in Current Protocols in Molecular Biology, John Wiley & Sons, N.Y. (1989), 6.3.1-6.3.6, which is incorporated by reference. Aqueous and non-aqueous methods are described in the references and either method can be used. The preferred hybridization conditions mentioned herein are as follows: 1) the low stringency hybridization condition is in 6× sodium chloride/sodium citrate (SSC) at about 45 °C, followed by two washes in 0.2× SSC, 0.1% SDS at least at 50 °C (for the low stringency condition, the temperature of the washes may be increased to 55 °C); 2) the medium stringency hybridization condition is in 6× SSC at about 45 °C, followed by one or more washes in 0.2× SSC, 0.1% SDS at 60 °C; 3) the high stringency hybridization condition is in 6× SSC at about 45 °C, followed by one or more washes in 0.2× SSC, 0.1% SDS at 65 °C; and preferably, 4) the extreme stringency hybridization condition is in 0.5 M sodium phosphate, 7% SDS at 65 °C, followed by one or more washes in 0.2× SSC, 0.1% SDS at 65 °C. The extreme stringency condition (4) is a preferred condition and the one that should be used unless otherwise stated.

The term "anti-tumor effect" refers to a biological effect that can be demonstrated by a variety of means, including but not limited to, for example, a decrease in tumor volume, a decrease in number of tumor cells, a decrease in tumor cell proliferation, or a decrease in tumor cell viability.

The terms "tumor" and "cancer" are used interchangeably herein and encompass solid and hematological tumors.

The terms "cancer" and "cancerous" refer to or describe a physiological disease in mammals that is typically characterized by unregulated cell growth. In certain embodiments, cancers suitable for treatment with the antibody of the present invention include gastric cancer, pancreatic cancer, or gastroesophageal junction cancer, including metastatic forms of such cancers.

The term "tumor" refers to all neoplastic cell growth and proliferation, whether malignant or benign, and all pre-cancerous and cancerous cells and tissues. The terms "cancer", "cancerous", and "tumor" are not mutually exclusive when referred to herein.

The term "pharmaceutical auxiliary material" refers to diluents, adjuvants (e.g., Freund's adjuvants (complete and incomplete)), excipients, carriers, stabilizers, or the like, that are administered with the active substance.

The term "antibody formulation" refers to a preparation in a form that allows the biological activity of an antibody as an active ingredient to be exerted effectively, and does not contain other components having unacceptable toxicity to a subject to which the formulation is to be administered. Such antibody formulations are generally sterile. Generally, the antibody formulation comprises a pharmaceutically acceptable excipient. A "pharmaceutically acceptable" excipient is an agent that can be reasonably administered to a mammal subject so that an effective dose of the active ingredient used in the formulation can be delivered to the subject. The concentration of the excipient is adapted to the mode of administration and may, for example, be acceptable for injection.

The term "anti-CLDN18.2/CD3 formulation", herein also referred to as the "antibody formulation of the present invention", refers to a preparation comprising an anti-CLDN18.2/CD3 protein as an active ingredient and a pharmaceutically acceptable excipient. The anti-CLDN18.2/CD3 protein, as the active ingredient, is suitable for therapeutic or prophylactic administration to a human or non-human animal after the anti-CLDN18.2/CD3 protein is combined with the pharmaceutically acceptable excipient. The antibody formulation of the present invention may be prepared, for example, as an aqueous liquid formulation, e.g., in a ready-to-use pre-filled syringe, or as a lyophilized formulation to be reconstituted (i.e., redissolved) by dissolution and/or suspension in a physiologically acceptable solution immediately prior to use. In some embodiments, the anti-CLDN18.2/CD3 protein formulation is in the form of a liquid formulation.

A "stable" antibody formulation refers to a formulation where the antibody retains an acceptable degree of physical and/or chemical stability after storage in specific conditions, after shaking and/or after repeated freezing-thawing. Although the antibody in the antibody formulation may not maintain 100% of its chemical structure after storage, shaking, or repeated freezing-thawing, the antibody formulation is considered "stable" when about 90%, about 95%, about 96%, about 97%, about 98%, or about 99% of the antibody structure or function is generally maintained. In some specific embodiments, the anti-CLDN18.2/CD3 protein formulation of the present invention exhibits undetectable antibody aggregation or degradation or chemical modification during manufacture, preparation, transportation, and long-term storage, and therefore there is little or even no loss of biological activity in the anti-CLDN18.2/CD3 protein, and the anti-CLDN18.2/CD3 protein exhibits high stability. In some embodiments, the anti-CLDN18.2/CD3 protein formulation of the present invention substantially retains its physical and chemical stability after storage, shaking, and/or repeated freezing-thawing. Preferably, the liquid formulation of the present invention may be stable at room temperature or at 40 °C for at least 2 weeks, and/or at 25 °C for at least 2 months, and/or at 2-8 °C for at least 24 months. Preferably, the liquid formulation of the present invention may be stable after shaking at room temperature in the dark at 650 r/min for 5 days and/or after 1-6 freezing-thawing repeats at -30 °C/room temperature.

A variety of analytical techniques are known in the art for determining the stability of proteins, see, e.g., Peptide and Protein Drug Delivery, 247-301, Vincent Lee Ed., Marcel Dekker, Inc., New York, N.Y., Pubs (1991) and Jones, A. Adv. Drug Delivery Rev. 10: 29-90 (1993). Stability can be determined at a selected temperature and for a selected storage time. For example, the storage time can be selected based on the expected shelf life of the formulation. Alternatively, an accelerated stability test can be adopted. In some embodiments, the stability test is performed by conducting various stress tests on the antibody formulation. These tests can represent extreme conditions that a formulated antibody formulation may encounter during manufacture, storage or transportation, and can also represent conditions that may accelerate the instability of the antibody in the antibody formulation during a non-manufacture, -storage or -transportation process. For example, the formulated anti-CLDN18.2/CD3 protein formulation may be filled into a glass vial to test the stability of the antibody under high temperature stress. For another example, the stability of the antibody may be tested by filling the formulated anti-CLDN18.2/CD3 protein formulation into a glass vial and shaking at room temperature in the dark at 650 r/min for 5 days. For another example, the stability of the antibody may be tested by filling the formulated anti-CLDN18.2/CD3 protein formulation into a glass vial and subjecting to 1-6 freezing-thawing repeats at -30 °C/room temperature. In one embodiment, a 1-day cryopreservation below -30 °C and a following thawing at room temperature constitute a freezing-thawing cycle.

The antibody can be considered to "maintain its physical stability" in the formulation if the formulation does not exhibit aggregation, precipitation, turbidity and/or denaturation, or exhibits very little aggregation, precipitation, turbidity, and/or denaturation after a period of storage, after a period of shaking, or after repeated freeze-thawing. Safety issues arise as the aggregation of antibodies in the formulation can potentially lead to an increased immune response in a patient. Accordingly, there is a need to minimize or prevent the aggregation of antibodies in the formulation. Light scattering methods can be used to determine visible aggregates in the formulation. SEC-HPLC can be used to determine soluble aggregates in the formulation. In addition, the stability of the formulation can be indicated by visually inspecting the appearance, color and/or clarity of the formulation, or by detecting the turbidity of the formulation by the OD_{350 nm} method, or by determining the purity of the formulation by the non-reduced CE-SDS method. In one embodiment, the stability of the formulation is measured by determining the percentage of antibody monomer in the formulation after storage at a specific temperature for a specific period of time, after shaking or after repeated freezing-thawing, wherein a higher percentage of antibody monomer in the formulation indicates a higher stability of the formulation.

An "acceptable degree" of physical stability may represent that at least about 90% of anti-CLDN18.2/CD3 protein monomer is detected in the formulation after storage at a specific temperature for a specific period of time, after shaking, or after repeated freezing-thawing. In some embodiments, an acceptable degree of physical stability represents at least about 90%, 91%, 92%, 93%, 94%, 95%, 96%, 97%, 98%, or 99% of anti-CLDN18.2/CD3 protein monomer after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When physical stability is assessed, the specific temperature at which the pharmaceutical formulation is stored may be any temperature from about -80 °C to about 45 °C, e.g., at about -80 °C, about -30 °C, about -20 °C, about 0 °C, about 4-8 °C, about 5 °C, about 25 °C, about 35 °C, about 37 °C, about 40 °C, about 42 °C, or about 45 °C. For example, a pharmaceutical formulation is considered stable if at least about 90%, 91%, 92%, 93%, 94%, 95%, 96%, 97%, 98%, or 99% of anti-CLDN18.2/CD3 protein monomer is detected after storage at about 40±2 °C for 1 month or 4 weeks. A pharmaceutical formulation is considered stable if at least about 90%, 91%, 92%, 93%, 94%, 95%, 96%, 97%, 98%, or 99% of anti-CLDN18.2/CD3 protein monomer is detected after storage at about 25 °C for 2 months. A pharmaceutical formulation is considered stable if at least about 90%, 91%, 92%, 93%, 94%, 95%, 96%, 97%, 98%, or 99% of anti-CLDN18.2/CD3 protein monomer is detected after storage at about 5 °C for 9 months.

The antibody can be considered to "maintain its chemical stability" in the formulation if the antibody in the formulation does not exhibit significant chemical changes after storage for a period of time, after a period of shaking, or after repeated freezing-thawing. Most of the chemical instability results from the formation of covalently modified forms of the antibody (e.g., charge variants of the antibody). Basic variants can be formed, for example, by aspartic acid isomerization, and N- and C-terminal modifications; acidic variants can be produced by deamidation, sialylation and saccharification. Chemical stability can be assessed by detecting and/or quantifying chemically altered forms of the antibody. For example, charge variants of the antibody in the formulation can be detected by cation exchange chromatography (CEX) or imaged capillary isoelectric focusing (iCIEF). In one embodiment, the stability of the formulation is measured by determining the percentage change in charge variants of the antibody in the formulation after storage at a specific temperature for a specific period of time, after shaking or after repeated freezing-thawing, wherein a smaller change indicates a higher stability of the formulation.

An "acceptable degree" of chemical stability may represent a percentage change in charge variants (e.g., main species, acidic species, or basic species) in the formulation not more than 40%, e.g., not more than 30% or not more than 20%, or a sum of percentage changes in charge variants (main species, acidic species, and basic species) not more than 60%, e.g., not more than 50% or not more than 30%, after storage at a specific temperature for a specific period of time, after a period of shaking, or after repeated freezing-thawing. In some embodiments, an acceptable degree of chemical stability may represent a percentage change in charge variants (main species) not more than about 50%, 40%, 30%, 20%, or 15%, or a sum of percentage changes in charge variants not more than about 60%, 50%, or 30%, after storage at a specific temperature for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When chemical stability is assessed, the temperature at which the pharmaceutical formulation is stored may be any temperature from about -80 °C to about 45 °C, e.g., at about -80 °C, about -30 °C, about -20 °C, about 0 °C, about 4-8 °C, about 5 °C, about 25 °C, or about 45 °C. For example, a pharmaceutical formulation may be considered stable if the percentage change in charge variants (main species) is less than about 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5%, or 0.1% after storage at 5 °C for 24 months. A pharmaceutical formulation may also be considered stable if the percentage change in charge variants (main species) is less than about 20%, 19%, 18%, 17%, 16%, 15%, 14%, 13%, 12%, 10%, 9%, 8%, 7%, 6%, 5%, 4%, 3%, 2%, 1%, 0.5%, or 0.1% after storage at 25 °C for 2 months. A pharmaceutical formulation may also be considered stable if the percentage change in charge variants (main species) is less than about 50%, 40%, 30%, 20%, 16%, 15%, 14%, 13%, 12%, 10%, 5%, or 4% after storage at 40 °C for 1 month.

The term "lyophilized formulation" refers to a composition obtained or obtainable by a lyophilization process of a liquid formulation. Preferably, it is a solid composition having a water content of less than 5%, preferably less than 3%.

The term "reconstituted formulation" refers to a liquid formulation obtained by dissolving and/or suspending a solid formulation (e.g., a lyophilized formulation) in a physiologically acceptable solution.

The term "room temperature" as used herein means a temperature from 15 °C to 30 °C, preferably from 20 °C to 27 °C, and more preferably 25 °C.

"Stress conditions" refer to environments that are chemically and/or physically unfavorable to antibody proteins and may result in unacceptable destabilization of the antibody proteins, e.g., high temperature, shaking and freezing-thawing. "High temperature stress" refers to storing the antibody formulation at room temperature or higher (e.g., 40±2 °C) for a period of time. The stability of the antibody formulation can be determined through a high temperature stress accelerated test.

As used herein, the term "parenteral administration" refers to administrations other than enteral and topical administrations, typically by injection or infusion, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In some embodiments, the stable anti-CLDN18.2/CD3 protein formulation of the present invention is administered parenterally to a subject. In one embodiment, the anti-CLDN18.2/CD3 protein formulation of the present invention is administered to a subject by subcutaneous, intradermal, intramuscular, or intravenous injection.

### I. Antibody Formulation

The present invention provides a stable liquid antibody formulation comprising: (i) an anti-CLDN18.2/CD3 antibody, (ii) a buffer, (iii) a stabilizer, and (iv) a surfactant, and optionally comprising a chelating agent. The antibody formulation has a pH of about 5.0-6.0. In one preferred embodiment, the liquid antibody formulation of the present invention is in the form of an injection.

### (i) Anti-CLDN18.2/CD3 antibody

In some embodiments, the anti-CLDN18.2/CD3 bispecific antibody of the present invention binds to CLDN18.2 (e.g., human CLDN18.2) with high affinity. In some embodiments, the antibody of the present invention specifically binds to CLDN18.2 (e.g., human CLDN18.2), but does not bind to CLDN18.1 (e.g., human CLDN18.1). In some embodiments, the antibody or the antigen-binding fragment thereof of the present invention has higher binding affinity for human CLDN18.2 than that of a known CLDN18.2 antibody, such as zolbetuximab (abbreviated as Zmab) antibody. In some embodiments, the anti-CLDN18.2×CD3 bispecific antibody of the present invention binds to CD3 (e.g., human CD3 or cynomolgus monkey CD3) with desired affinity (e.g., lower). In some embodiments, the antibody of the present invention is capable of binding to both human CD3 and cynomolgus monkey CD3. In some embodiments, the affinity of the antibody is determined by bio-layer interferometry or surface plasmon resonance.

In some embodiments, the anti-CLDN18.2/CD3 bispecific antibody of the present invention is capable of binding to CLDN18.2 on the surface of tumor cells, as well as CD3 on the surface of effector cells. In some embodiments, the binding is determined by flow cytometry.

In some embodiments, the bispecific antibody of the present invention comprises a first antigen-binding domain and a second antigen-binding domain, wherein the first antigen-binding domain specifically binds to CLDN18.2, and the second antigen-binding domain specifically binds to CD3.

In a preferred embodiment of the present invention, the first antigen-binding domain comprises 3 complementarity determining regions from a heavy chain variable region (A1-HCDRs), i.e., A1-HCDR1, A1-HCDR2, and A1-HCDR3.

In a preferred embodiment of the present invention, the first antigen-binding domain comprises 3 complementarity determining regions from a light chain variable region (A1-LCDRs), i.e., A1-LCDR1, A1-LCDR2, and A1-LCDR3.

In some embodiments, the first antigen-binding domain comprises 3 complementarity determining regions from a heavy chain variable region (A1-HCDRs) and 3 complementarity determining regions from a light chain variable region (A1-LCDRs).

In some aspects, the first antigen-binding domain comprises a heavy chain variable region (A1-VH). In some aspects, the first antigen-binding domain comprises a light chain variable region (A1-VL). In some aspects, the first antigen-binding domain comprises a heavy chain variable region and a light chain variable region. In some embodiments, the heavy chain variable region comprises 3 complementarity determining regions from the heavy chain variable region (A1-HCDRs), i.e., HCDR1, HCDR2, and HCDR3. In some embodiments, the light chain variable region comprises 3 complementarity determining regions from the light chain variable region (A1-LCDRs), i.e., LCDR1, LCDR2, and LCDR3.

In some embodiments, the A1-VH
(i) comprises or consists of an amino acid sequence having at least 90%, 91%, 92%, 93%, 94%, 95%, 96%, 97%, 98%, or 99% identity to the amino acid sequence selected from SEQ ID NO: 4; or
(ii) comprises or consists of the amino acid sequence selected from SEQ ID NO: 4; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 4, wherein preferably, the amino acid changes do not occur in the CDRs.

In some embodiments, the A1-VL
(i) comprises or consists of an amino acid sequence having at least 90%, 91%, 92%, 93%, 94%, 95%, 96%, 97%, 98%, or 99% identity to the amino acid sequence selected from SEQ ID NO: 9; or
(ii) comprises or consists of the amino acid sequence selected from SEQ ID NO: 9; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 9, wherein preferably, the amino acid changes do not occur in the CDRs.

In a preferred embodiment of the present invention, the second antigen-binding domain comprises 3 complementarity determining regions from a heavy chain variable region (A2-HCDRs), i.e., A2-HCDR1, A2-HCDR2, and A2-HCDR3.

In a preferred embodiment of the present invention, the second antigen-binding domain comprises 3 complementarity determining regions from a light chain variable region (A2-LCDRs), i.e., A2-LCDR1, A2-LCDR2, and A2-LCDR3.

In some embodiments, the second antigen-binding domain comprises 3 complementarity determining regions from a heavy chain variable region (A2-HCDRs) and 3 complementarity determining regions from a light chain variable region (A2-LCDRs).

In some aspects, the second antigen-binding domain comprises a heavy chain variable region (A2-VH). In some aspects, the second antigen-binding domain comprises a light chain variable region (A2-VL). In some aspects, the second antigen-binding domain comprises a heavy chain variable region and a light chain variable region. In some embodiments, the heavy chain variable region comprises 3 complementarity determining regions from the heavy chain variable region (A2-HCDRs), i.e., HCDR1, HCDR2, and HCDR3. In some embodiments, the light chain variable region comprises 3 complementarity determining regions from the light chain variable region (A2-LCDRs), i.e., LCDR1, LCDR2, and LCDR3.

In some embodiments, the A2-VH
(i) comprises or consists of an amino acid sequence having at least 90%, 91%, 92%, 93%, 94%, 95%, 96%, 97%, 98%, or 99% identity to the amino acid sequence selected from SEQ ID NO: 22, 30, or 32; or
(ii) comprises or consists of the amino acid sequence selected from SEQ ID NO: 22, 30, or 32; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 22, 30, or 32, wherein preferably, the amino acid changes do not occur in the CDRs.

In some embodiments, the A2-VL
(i) comprises or consists of an amino acid sequence having at least 90%, 91%, 92%, 93%, 94%, 95%, 96%, 97%, 98%, or 99% identity to the amino acid sequence selected from SEQ ID NO: 27; or
(ii) comprises or consists of the amino acid sequence selected from SEQ ID NO: 27; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 27, wherein preferably, the amino acid changes do not occur in the CDRs.

In some embodiments, the 3 complementarity determining regions from the A2-VH (A2-HCDRs) of the present invention, i.e., A2-HCDR1, A2-HCDR2, and A2-HCDR3, are
(i) three complementarity determining regions A2-HCDR1, A2-HCDR2, and A2-HCDR3 contained in a VH set forth in SEQ ID NO: 22, 30, or 32, or
(ii) relative to the sequences in (i), sequences comprising a total of at least one and no more than 5, 4, 3, 2, or 1 amino acid change (preferably amino acid replacement, and more preferably conservative replacement) in the three A2-HCDRs.

In some embodiments, the 3 complementarity determining regions from the A2-VL (A2-LCDRs) of the present invention, i.e., A2-LCDR1, A2-LCDR2, and A2-LCDR3, are
(i) three complementarity determining regions A2-LCDR1, A2-LCDR2, and A2-LCDR3 contained in an A2-VL set forth in SEQ ID NO: 27, or
(ii) relative to the sequences in (i), sequences comprising a total of at least one and no more than 5, 4, 3, 2, or 1 amino acid change (preferably amino acid replacement, and more preferably conservative replacement) in the three A2-LCDRs.

In some embodiments, the A2-HCDR1 comprises or consists of the amino acid sequence set forth in SEQ ID NO: 19, or the A2-HCDR1 comprises an amino acid sequence having one, two, or three changes (preferably amino acid replacements, and more preferably conservative replacements) compared with the amino acid sequence set forth in SEQ ID NO: 19.

In some embodiments, the A2-HCDR2 comprises or consists of the amino acid sequence set forth in SEQ ID NO: 20 or 31, or the A2-HCDR2 comprises an amino acid sequence having one, two, or three changes (preferably amino acid replacements, and more preferably conservative replacements) compared with the amino acid sequence set forth in SEQ ID NO: 20 or 31.

In some embodiments, the A2-HCDR3 comprises or consists of the amino acid sequence set forth in SEQ ID NO: 21 or 29, or the A2-HCDR3 comprises an amino acid sequence having one, two, or three changes (preferably amino acid replacements, and more preferably conservative replacements) compared with the amino acid sequence set forth in SEQ ID NO: 21 or 29.

In some embodiments, the A2-LCDR1 comprises or consists of the amino acid sequence set forth in SEQ ID NO: 24, or the A2-LCDR1 comprises an amino acid sequence having one, two, or three changes (preferably amino acid replacements, and more preferably conservative replacements) compared with the amino acid sequence set forth in SEQ ID NO: 24.

In some embodiments, the A2-LCDR2 comprises or consists of the amino acid sequence set forth in SEQ ID NO: 25, or the A2-LCDR2 comprises an amino acid sequence having one, two, or three changes (preferably amino acid replacements, and more preferably conservative replacements) compared with the amino acid sequence set forth in SEQ ID NO: 25.

In some embodiments, the A2-LCDR3 comprises or consists of the amino acid sequence set forth in SEQ ID NO: 26, or the A2-LCDR3 comprises an amino acid sequence having one, two, or three changes (preferably amino acid replacements, and more preferably conservative replacements) compared with the amino acid sequence set forth in SEQ ID NO: 26.

In some embodiments, the bispecific antibody of the present invention further comprises a heavy chain constant region. In some embodiments, the bispecific antibody of the present invention further comprises a light chain constant region. In some embodiments, the bispecific antibody of the present invention further comprises a heavy chain constant region and a light chain constant region. In some embodiments, the bispecific antibody of the present invention comprises 2 heavy chain constant regions, wherein one of the heavy chain constant regions A1-HC is linked to the heavy chain variable region A1-VH of the first antigen domain to form a part of a heavy chain binding to CDLN18.2, and the other heavy chain constant region A2-HC is linked to the heavy chain variable region A2-VH of the second antigen-binding domain to form a part of a heavy chain binding to CD3. In some embodiments, the bispecific antibody of the present invention comprises 2 light chain constant regions, wherein one of the light chain constant regions A1-LC is linked to the light chain variable region A1-VL of the first antigen domain to form a part of a light chain binding to CLDN18.2, and the other light chain constant region A2-LC is linked to the light chain variable region A2-VL of the second antigen-binding domain to form a part of a light chain binding to CD3. In one embodiment, the bispecific antibody of the present invention comprises A1-HC, A1-LC, A2-HC, and A2-LC. In some embodiments, the A1-HC may be the same as or different from the A2-HC. In some embodiments, the A1-LC may be the same as or different from the A2-LC. In some embodiments, the A1-HC is different from the A2-HC, and the A1-LC is different from the A2-LC.

In some embodiments, the heavy chain constant region HC of the present invention is a heavy chain constant region of IgG1, IgG2, IgG3, or IgG4, preferably a heavy chain constant region of IgG1, such as a heavy chain constant region of wild-type IgG1 or a heavy chain constant region of mutated IgG1 (e.g., IgG1LALA). In some embodiments, the antibody light chain constant region LC of the present invention is a lambda or Kappa light chain constant region.

In some preferred embodiments, the heavy chain constant region HC of the present invention
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 5 or 23;
(ii) comprises or consists of the amino acid sequence selected from SEQ ID NO: 5 or 23; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 5 or 23.

In some preferred embodiments, the heavy chain constant region HC of the present invention
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 33 or 34;
(ii) comprises or consists of the amino acid sequence selected from SEQ ID NO: 33 or 34; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 33 or 34.

In some embodiments, the antibody light chain constant region LC of the present invention
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 10 or 28;
(ii) comprises or consists of the amino acid sequence selected from SEQ ID NO: 10 or 28; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 10 or 28.

In some specific embodiments of the present invention, the bispecific antibody of the present invention comprises a first antigen-binding domain and a second antigen-binding domain, wherein
the first antigen-binding domain specifically binds to CLDN18.2, and the second antigen-binding domain specifically binds to CD3, wherein the first antigen-binding domain comprises three complementarity determining regions A1-HCDR1, A1-HCDR2, and A1-HCDR3 contained in an A1-VH set forth in SEQ ID NO: 4, and three complementarity determining regions A1-LCDR1, A1-LCDR2, and A1-LCDR3 contained in a VL set forth in SEQ ID NO: 9;
the second antigen-binding domain comprises three complementarity determining regions A2-HCDR1, A2-HCDR2, and A2-HCDR3 contained in an A2-VH set forth in SEQ ID NO: 22, 30, or 32, and three complementarity determining regions A2-LCDR1, A2-LCDR2, and A2-LCDR3 contained in an A2-VL set forth in SEQ ID NO: 27.

In some specific embodiments of the present invention, the bispecific antibody of the present invention comprises a first antigen-binding domain and a second antigen-binding domain, wherein the first antigen-binding domain specifically binds to CLDN18.2, and the second antigen-binding domain specifically binds to CD3, wherein
the first antigen-binding domain comprises A1-HCDR1, A1-HCDR2, and A1-HCDR3 set forth in the amino acid sequences of SEQ ID NOs: 1, 2, and 3, respectively, and A1-LCDR1, A1-LCDR2, and A1-LCDR3 set forth in the amino acid sequences of SEQ ID NOs: 6, 7, and 8, respectively; and
the second antigen-binding domain comprises:
   (i) A2-HCDR1, A2-HCDR2, and A2-HCDR3 set forth in the amino acid sequences of SEQ ID NOs: 19, 20, and 21, respectively, and LCDR1, LCDR2, and LCDR3 set forth in the amino acid sequences of SEQ ID NOs: 24, 25, and 26, respectively; or
   (ii) A2-HCDR1, A2-HCDR2, and A2-HCDR3 set forth in the amino acid sequences of SEQ ID NOs: 19, 20, and 29, respectively, and LCDR1, LCDR2, and LCDR3 set forth in the amino acid sequences of SEQ ID NOs: 24, 25, and 26, respectively; or
   (iii) A2-HCDR1, A2-HCDR2, and A2-HCDR3 set forth in the amino acid sequences of SEQ ID NOs: 19, 31, and 21, respectively, and LCDR1, LCDR2, and LCDR3 set forth in the amino acid sequences of SEQ ID NOs: 24, 25, and 26, respectively.

In some specific embodiments of the present invention, the bispecific antibody of the present invention comprises a first antigen-binding domain and a second antigen-binding domain, wherein the first antigen-binding domain specifically binds to CLDN18.2, and the second antigen-binding domain specifically binds to CD3, wherein
the first antigen-binding domain comprises an A1-VH comprising or consisting of the amino acid sequence set forth in SEQ ID NO: 4 or an amino acid sequence having at least 90%, 91%, 92%, 93%, 94%, 95%, 96%, 97%, 98%, or 99% identity thereto, and an A1-VL comprising or consisting of the amino acid sequence set forth in SEQ ID NO: 9 or an amino acid sequence having at least 90% identity thereto;
the second antigen-binding domain comprises an A2-VH comprising or consisting of the amino acid sequence set forth in SEQ ID NO: 22, 30, or 32 or an amino acid sequence having at least 90% identity thereto, and an A2-VL comprising or consisting of the amino acid sequence set forth in SEQ ID NO: 27 and an amino acid sequence having at least 90%, 91%, 92%, 93%, 94%, 95%, 96%, 97%, 98%, or 99% identity thereto.

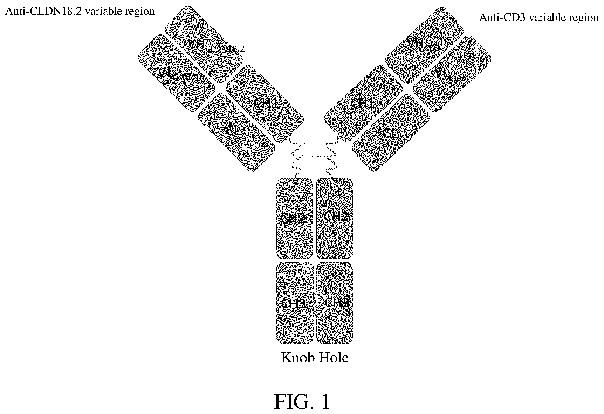

In some specific embodiments of the present invention, the bispecific antibody of the present invention is a bispecific antibody with an IgG-like structure, i.e., it comprises a part of a heavy chain and a light chain binding to CLDN18.2 and a part of a heavy chain and a light chain binding to CD3. In some embodiments, there are knob-into-hole sequences in the CH3 regions of the two heavy chains (Shane Atwell et al., Journal of Molecular Biology, 1997; A. Margaret Merchant et al., Nature Biotechnology, 1998), thus forming a knob-into-hole structure. In some embodiments, the structure of the bispecific antibody of the present invention is shown in FIG. 1.

In some embodiments, the part of the heavy chain binding to CLDN18.2
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 37;
(ii) comprises or consists of the amino acid sequence selected from SEQ ID NO: 37; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 37.

In some embodiments, the part of the light chain binding to CLDN18.2
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 38;
(ii) comprises or consists of the amino acid sequence selected from SEQ ID NO: 38; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 38.

In some embodiments, the part of the heavy chain binding to CD3
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 39, 41, or 42;
(ii) comprises or consists of the amino acid sequence selected from SEQ ID NO: 39, 41, or 42; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 39, 41, or 42.

In some embodiments, the part of the light chain binding to CD3
(i) comprises or consists of an amino acid sequence having at least 85%, 90%, 91%, 92%, 93%, 94%, 95%, 96%, 97%, 98%, or 99% identity to the amino acid sequence selected from SEQ ID NO: 40;
(ii) comprises or consists of the amino acid sequence selected from SEQ ID NO: 40; or
(iii) comprises or consists of an amino acid sequence having one or more (preferably no more than 20 or 10, and more preferably no more than 5, 4, 3, 2, or 1) amino acid changes (preferably amino acid replacements, and more preferably conservative amino acid replacements) compared with the amino acid sequence selected from SEQ ID NO: 40.

In one embodiment of the present invention, the amino acid change described herein includes amino acid replacement, insertion, or deletion. Preferably, the amino acid change described herein is an amino acid replacement, preferably a conservative replacement.

In a preferred embodiment, the amino acid change described herein occurs in a region outside the CDR (e.g., in FR). More preferably, the amino acid change described herein occurs in a region outside the heavy chain variable region and/or outside the light chain variable region. In some embodiments, the amino acid changes described herein occur in the Fc region of the antibody heavy chain constant region. In a preferred embodiment, the amino acid changes in the Fc region attenuate or delete the ADCC and/or CDC effect of the antibody.

In some embodiments, the replacement is a conservative replacement. A conservative replacement refers to the replacement of an amino acid by another amino acid of the same class, e.g., the replacement of an acidic amino acid by another acidic amino acid, the replacement of a basic amino acid by another basic amino acid, or the replacement of a neutral amino acid by another neutral amino acid. Exemplary replacements are shown in the table below:

| Original residue | Exemplary replacement | Preferred conservative amino acid replacement |
|---|---|---|
| Ala (A) | Val, Leu, Ile | Val |
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu, Val, Met, Ala, Phe, Nle | Leu |
| Leu (L) | Nle, Ile, Val, Met, Ala, Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr(T) | Val, Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Nle | Leu |

In certain embodiments, the replacement occurs in the CDRs of the antibody. Generally, the obtained variant has modifications (e.g., improvements) in certain biological properties (e.g., increased affinity) relative to the parent antibody and/or will have certain substantially retained biological properties of the parent antibody. Exemplary replacement variants are affinity-matured antibodies.

In certain embodiments, one or more amino acid modifications may be introduced into the Fc region of the antibody provided herein, thereby producing an Fc region variant to alter one or more functional properties of the antibody, such as serum half-life, complement fixation, complement-dependent cytotoxicity, Fc receptor binding and/or antibody-dependent cell-mediated cytotoxicity. The Fc region variant may comprise a human Fc region sequence (such as human IgG1, IgG2, IgG3, or IgG4 Fc region) comprising an amino acid change (such as replacement) at one or more amino acid positions.

In certain embodiments, antibodies modified by cysteine engineering may need to be produced, such as "sulfo-MAb", wherein one or more residues of the antibodies are replaced by cysteine residues.

In certain embodiments, the antibody provided herein can be further modified to comprise other non-protein portions known in the art and readily available. Suitable portions for antibody derivatization include, but are not limited to, water-soluble polymers. Non-limiting examples of water-soluble polymers include, but are not limited to, polyethylene glycol (PEG), ethylene glycol/propylene glycol copolymer, carboxymethyl cellulose, glucan, polyvinyl alcohol, polyvinylpyrrolidone, poly-1,3-dioxane, poly-1,3,6-trioxane, ethylene/maleic anhydride copolymer, polyamino acid (homopolymer or random copolymer), and glucan or poly(n-vinylpyrrolidone)polyethylene glycol, propylene glycol homopolymer, polypropylene oxide/ethylene oxide copolymer, polyoxyethylated polyol (such as glycerol), polyvinyl alcohol, and mixtures thereof.

In some embodiments, in the bispecific antibody of the present invention, the first antigen-binding domain specifically binding to CLDN18.2 or the combination of the heavy chain and the light chain is fully human, and the second antigen-binding domain specifically binding to CD3 or the combination of the heavy chain and the light chain is humanized.

In a preferred embodiment, the anti-CLDN18.2/CD3 bispecific antibody in the antibody formulation of the present invention is the anti-CLDN18.2/CD3 bispecific antibody disclosed in PCT Application No. PCT/CN2021/121286 (International application date: Sep. 28, 2021).

The amount of antibody or antigen-binding fragment thereof in the antibody formulation of the present invention may vary with the specific desired properties of the formulation, the specific environment, and the specific purpose for which the formulation is used. In some embodiments, the antibody formulation is a liquid formulation, which may comprise about 0.1-100 mg/mL, preferably about 0.1-10 mg/mL, e.g., about 0.5 mg/mL, 1 mg/mL, 2 mg/mL, 3 mg/mL, 4 mg/mL, or 5 mg/mL anti-CLDN18.2/CD3 bispecific antibody.

### (ii) Buffer

Buffers are reagents that can control the pH of a solution within an acceptable range. In some embodiments, the buffer used in the formulation of the present invention may control the pH of the formulation of the present invention to a pH range of about 5.0-6.0, e.g., a pH of about 5.0-5.5. In some specific embodiments, the antibody formulation of the present invention has a pH of about 5.0, 5.1, 5.2, 5.3, 5.4, 5.5, 5.6, 5.7, or 5.8. For example, the antibody formulation of the present invention has a pH of 5.2±0.2 or 5.5±0.2, preferably a pH of 5.2.

In some embodiments, the formulation of the present invention comprises a buffer system selected from: a histidine-histidine hydrochloride buffer system, a citric acid-sodium citrate buffer system, an acetic acid-sodium acetate buffer system, and a phosphate buffer system, preferably a histidine-histidine hydrochloride buffer system. In some embodiments, the buffer used in the formulation of the present invention is a histidine buffer, particularly a buffer system consisting of histidine and histidine hydrochloride. In some embodiments, the concentration of histidine in the histidine buffer of the present invention is about 0.01-10 mg/mL mM, particularly about 0.1-1 mg/mL, e.g., about 0.1 mg/mL, 0.2 mg/mL, 0.3 mg/mL, 0.4 mg/mL, 0.5 mg/mL, or 1 mg/mL. In one embodiment, the formulation of the present invention comprises about 0.18 mg/mL histidine. In another embodiment, the histidine buffer used in the formulation of the present invention consists of, for example, about 0.18 mg/mL histidine and about 1.84 mg/mL histidine hydrochloride.

### (iii) Stabilizer

Suitable stabilizers for use in the present invention can be selected from saccharides, polyols and amino acids and a combination thereof. Saccharides that may be used as a stabilizer include, but are not limited to, sucrose, trehalose, maltose, and a combination thereof. Polyols that may be used as a stabilizer include, but are not limited to, sorbitol, mannitol, and a combination thereof. Amino acids that may be used as a stabilizer include, but are not limited to, arginine, arginine hydrochloride, methionine, glycine, proline, and a combination thereof.

For example, in some embodiments, the stabilizer comprises one or more selected from:
- a polyol selected from sorbitol, mannitol, or a combination thereof, at about 10-100 mg/mL, preferably 20-40 mg/mL, e.g., 25 mg/mL; or 40-60 mg/mL, e.g., 50 mg/mL;
- a saccharide selected from sucrose, trehalose, maltose, or a combination thereof, for example, at about 10-100 mg/mL, preferably 30-60 mg/mL, e.g., 40 mg/mL; and
- an amino acid selected from arginine hydrochloride, methionine, glycine, proline, and a combination thereof, for example, at 20-200 mM, such as about 50-110 mM, e.g., 70-100 mM, particularly about 80-90 mM.

In one embodiment, the liquid formulation of the present invention comprises sucrose as the stabilizer. The amount of sucrose in the liquid formulation of the present invention may be about 10-100 mg/mL, preferably 30-60 mg/mL, e.g., 40 mg/mL.

In one embodiment, the liquid formulation of the present invention comprises sorbitol as the stabilizer. The amount of sorbitol in the liquid formulation of the present invention may be about 10-100 mg/mL, preferably 20-40 mg/mL, e.g., 25 mg/mL; or 40-60 mg/mL, e.g., 50 mg/mL.

In one embodiment, the liquid formulation of the present invention comprises arginine as the stabilizer. The amount of arginine in the liquid formulation of the present invention may be about 50-110 mM, e.g., 70-100 mM, particularly about 80-90 mM, e.g., about 17.91 mg/mL arginine hydrochloride.

In one embodiment, the liquid formulation of the present invention comprises a combination of sorbitol and arginine as the stabilizer. In the combination, sorbitol may be present in an amount of about 10-60 mg/mL, preferably 15-40 mg/mL, e.g., 20-35 mg/mL, e.g., about 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, or 30 mg/mL. In the combination, arginine may be present in an amount of about 70-100 mM, particularly about 85 mM. Preferably, the liquid formulation of the present invention comprises about 20-30 mg/mL sorbitol and about 15-20 mg/mL arginine hydrochloride. More preferably, the liquid formulation of the present invention comprises about 25 mg/mL sorbitol and about 17.91 mg/mL arginine hydrochloride.

In one embodiment, the liquid formulation of the present invention comprises a combination of sucrose and arginine as the stabilizer. In the combination, sucrose may be present in an amount of about 10-60 mg/mL, preferably 20-50 mg/mL, e.g., 30-40 mg/mL, e.g., about 30 mg/mL, 32 mg/mL, 34 mg/mL, 36 mg/mL, 38 mg/mL, 40 mg/mL, 42 mg/mL, 44 mg/mL, 46 mg/mL, 48 mg/mL, or 50 mg/mL. In the combination, arginine may be present in an amount of about 70-100 mM, particularly about 85 mM. Preferably, the liquid formulation of the present invention comprises about 30-50 mg/mL sucrose and about 15-20 mg/mL arginine hydrochloride. More preferably, the liquid formulation of the present invention comprises about 40 mg/mL sucrose and about 17.91 mg/mL arginine hydrochloride.

In one embodiment, the liquid formulation of the present invention comprises sorbitol as the sole stabilizer. In this embodiment, the amount of sorbitol in the liquid formulation of the present invention may be about 30-70 mg/mL, e.g., 40-60 mg/mL. For example, sorbitol may be present in an amount of about 30 mg/mL, 35 mg/mL, 40 mg/mL, 45 mg/mL, 50 mg/mL, 55 mg/mL, 60 mg/mL, 65 mg/mL, or 70 mg/mL, preferably about 50 mg/mL.

### (iv) Surfactant

As used herein, the term "surfactant" refers to an organic substance with an amphiphilic structure; that is, the structure is composed of groups with opposite solubility tendencies, typically an oil-soluble hydrocarbon chain and a water-soluble ionic group.

In one embodiment, the surfactant in the liquid formulation of the present invention is a non-ionic surfactant, e.g., alkyl poly(ethylene oxide). Specific non-ionic surfactants that may be contained in the formulation of the present invention include, for example, polysorbates such as polysorbate-20, polysorbate-80, polysorbate-60 or polysorbate-40, poloxamer, and the like. In one preferred embodiment, the liquid formulation of the present invention comprises polysorbate-80 as the surfactant.

In some embodiments, surfactants that may be used in the liquid formulation of the present invention include, but are not limited to, polysorbate surfactants (e.g., polysorbate 80 and polysorbate 20), poloxamer, and polyethylene glycol.

The amount of surfactant in the antibody formulation of the present invention may vary with the specific desired properties of the formulation, the specific environment, and the specific purpose for which the formulation is used. In some preferred embodiments, the formulation may comprise about 0.01-5 mg/mL, preferably about 0.1-1 mg/mL, e.g., about 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL surfactant, particularly polysorbate-80, preferably about 0.2 mg/mL polysorbate-80.

### (v) Chelating agent

As used herein, the term "chelating agent" refers to a compound that is capable of forming a chelate with a central atom, and the stability of the complex is greatly increased due to the formation of the chelate.

In one embodiment, the chelate in the liquid formulation of the present invention is a carboxylic acid chelating agent. In one embodiment, the chelating agent is selected from edetate disodium, nitrilotriacetic acid, diethylenetriaminepentaacetic acid, citric acid, tartaric acid, gluconic acid, hydroxyethylethylenediaminetriacetic acid, and dihydroxyethylglycine. In one embodiment, the chelating agent is selected from edetate disodium.

In one embodiment, the concentration of the chelating agent in the liquid antibody formulation of the present invention is about 0.005-0.05 mg/mL. In one embodiment, the concentration of the chelating agent in the liquid antibody formulation of the present invention is about 0.008-0.018 mg/mL, e.g., about 0.008 mg/mL, 0.009 mg/mL, 0.010 mg/mL, 0.012 mg/mL, 0.014 mg/mL, or 0.018 mg/mL, preferably 0.01 mg/mL.

### (vi) Other excipients

The liquid antibody formulation of the present invention optionally comprises other excipients. Such other excipients include, for example, antimicrobials, antistatic agents, antioxidants, chelating agents, gelatin, and the like. These and other known pharmaceutical excipients and/or additives suitable for use in the formulation of the present invention are well known in the art, for example, as listed in "The Handbook of Pharmaceutical Excipients, 4th Ed., edited by Rowe et al., American Pharmaceuticals Association (2003); and Remington: the Science and Practice of Pharmacy, 21st Ed., edited by Gennaro, Lippincott Williams & Wilkins (2005)".

### II. Preparation of Formulation

The present invention provides a stable formulation comprising an anti-CLDN18.2/CD3 antibody. The anti-CLDN18.2/CD3 antibody used in the formulation of the present invention may be prepared using techniques known in the art for the production of antibodies. For example, the antibody may be recombinantly prepared. In one preferred embodiment, the antibody of the present invention is recombinantly prepared in 293 cells or CHO cells.

The use of antibodies as active ingredients in drugs is now very common. Techniques for purifying therapeutic antibodies to pharmaceutical grade are well known in the art. For example, Tugcu et al. (Maximizing productivity of chromatography steps for purification of monoclonal antibodies, Biotechnology and Bioengineering, 99 (2008) 599-613) described a monoclonal antibody three-column purification method in which ion exchange chromatography (anionic IEX and/or cationic CEX chromatography) is used after a protein A capture step. Kelley et al. (Weak partitioning chromatography for anion exchange purification of monoclonal antibodies, Biotechnology and Bioengineering, 101 (2008) 553-566) described a two-column purification method in which a weak partitioning anion exchange resin is used after protein A affinity chromatography.

Generally, antibodies recombinantly produced may be purified using conventional purification methods to provide a drug substance with sufficient reproducibility and proper purity for the formulation of antibody formulations. For example, after the antibody is secreted from the recombinant expression cells into the culture medium, the supernatant of the expression system may be concentrated using a commercially available protein concentration filter, e.g., an Amicon ultrafiltration device. Then the antibody may be purified by methods such as chromatography, dialysis and affinity purification. Protein A is suitable as an affinity ligand for the purification of IgG1, IgG2 and IgG4 antibodies. Other antibody purification methods, such as ion exchange chromatography, may also be used. After the antibody with sufficient purity is obtained, a formulation comprising the antibody may be prepared according to methods known in the art.

For example, the preparation may be performed by the following steps: (1) removing impurities such as cells from fermentation broth by centrifuging and clarifying after the fermentation to give a supernatant; (2) capturing an antibody using affinity chromatography (e.g., a protein A column with specific affinity for IgG1, IgG2, and IgG4 antibodies); (3) inactivating viruses; (4) purifying (usually CEX cation exchange chromatography may be adopted) to remove impurities in a protein; (5) filtering the viruses (to reduce the virus titer by, e.g., more than 4 log10); and (6) ultrafiltering/diafiltering (which may be used to allow the protein to be exchanged into a formulation buffer that is favorable for its stability and concentrated to a suitable concentration for injection). See, e.g., B. Minow, P. Rogge, K. Thompson, BioProcess International, Vol. 10, No. 6, 2012, pp. 48-57.

### III. Analytical Method of Formulation

Biologies stability studies typically include real-time stability studies in actual storage conditions (long-term stability studies), accelerated stability studies and forced condition studies. For the stability studies, the study conditions are explored and optimized according to the purpose and the properties of the product; stability study protocols, such as long-term, accelerated, and/or forced condition studies and the like, should be established according to various influencing factors (such as temperature, repeated freezing-thawing, vibration, and the like). Accelerated and forced condition studies are beneficial to understanding the stability of the product in short-term deviations from storage conditions and in extreme conditions, and provide supporting data for the determination of the shelf life and storage conditions.

During the storage, shaking or repeated freezing-thawing of antibody formulations, antibodies may undergo aggregation, degradation or chemical modification, resulting in antibody heterogeneity (including size heterogeneity and charge heterogeneity), aggregates and fragments, etc., which may affect the quality of the antibody formulations. Accordingly, it is necessary to monitor the stability of antibody formulations.

Various methods are known in the art for testing the stability of antibody formulations. For example, the purity of the antibody formulation can be analyzed and the aggregation level of the antibody can be evaluated by methods such as reduced CE-SDS, non-reduced CE-SDS, and SEC-HPLC; charge variants in the antibody formulation can be analyzed by ion exchange chromatography (IEX) and the like. In addition, the stability of the formulation can be determined quickly by visually inspecting the appearance of the formulation. The change in turbidity of the formulation can also be detected by the OD_{350 nm} method, which gives information about the amount of soluble and insoluble aggregates. In addition, the change in protein content in the formulation can be detected by ultraviolet spectrophotometry (UV method).

Non-reduced CE-SDS is a method for determining the purity of antibodies using a capillary as a separation channel. In CE-SDS, protein migration is driven by the surface charge caused by SDS binding, which is proportional to the molecular weight of the protein. Since all SDS-protein complexes have similar mass-to-charge ratios, electrophoretic separation based on the size or hydrodynamic radius of the molecules can be achieved in the molecular sieve gel matrix of the capillary. This method has been widely used to monitor the purity of denatured intact antibodies. Generally, in non-reduced CE-SDS, the test sample is mixed with an SDS sample buffer and iodoacetamide. Then, the mixture can be incubated at 68-72 °C for about 10-15 min and cooled to room temperature before the supernatant is centrifuged for analysis. The protein migration is detected using an ultraviolet detector to give an electropherogram. The purity of the antibody formulation can be calculated as the percentage of the IgG main peak area to the sum of all peak areas. For further description of CE-SDS, see, e.g., Richard R. et al., Application of CE SDS gel in development of biopharmaceutical antibody-based products, Electrophoresis, 2008, 29, 3612-3620.

Size exclusion chromatography-high performance liquid chromatography (SEC-HPLC) is another important method for the standardization and quality control of antibodies. In this method, molecules are separated mainly based on the differences in their size or hydrodynamic radius. Antibodies can be separated into three main forms by SEC-HPLC: high-molecular-weight species (HMMS), main peak (mainly antibody monomer), and low-molecular-weight species (LMMS). The purity of the antibody can be calculated as the percentage of the main peak area to the sum of all peak areas on the chromatogram. The percentage of antibody monomer in the formulation can be measured by SEC-HPLC, which gives information about the content of soluble aggregates and splices. For further description of SEC-HPLC, see, e.g., J. Pharm. Scien., 83: 1645-1650, (1994); Pharm. Res., 11: 485 (1994); J. Pharm. Bio. Anal., 15: 1928 (1997); J. Pharm. Bio. Anal., 14: 1133-1140 (1986). In addition, see also, e.g., R. Yang et al., High resolution separation of recombinant monoclonal antibodies by size exclusion ultra-high performance liquid chromatography (SE-UHPLC), Journal of Pharmaceutical and Biomedical Analysis (2015), http://dx.doi.org/10.1016/j.jpba.2015.02.032; and Alexandre Goyon et al., Protocols for the analytical characterization of therapeutic monoclonal antibodies, I-Non-denaturing chromatographic techniques, Journal of Chromatography, http://dx.doi.org/10.1016/j.jchromb.2017.05.010.

Accelerated stability studies can be used to check the stability of products, which facilitates the screening of stable pharmaceutical formulations. For example, formulation samples can be placed at an elevated temperature, e.g., about 40±2 °C or 25±2 °C for an accelerated stability study. In addition, the shaking test or repeated freezing-thawing test can be conducted to test the stability properties of the product. For example, the shaking test is conducted at room temperature in the dark at 650 r/min for 1-5 days. For example, the repeated freezing-thawing test can be conducted by a cycle of a one-day cryopreservation below -30 °C and a following thawing at room temperature, wherein 1-6 repeated freezing-thawing cycles can be performed. The test indexes for product stability may include appearance, visible particles, protein content, turbidity, purity (SEC-HPLC and non-reduced CE-SDS) and charge variants (iCIEF and CEX-HPLC). In addition, the efficacy or biological activity of the antibody can be detected. For example, the ability of the antibody in the formulation to bind to its antigenic molecule (TIGIT) can be tested. Various methods are known to those skilled in the art for quantifying the specific binding of an antibody to an antigen, such as immunoassay, e.g., ELISA.

### Abbreviations

| Abbreviation | Full name |
|---|---|
| CE-SDS | Sodium dodecyl sulfate capillary electrophoresis |
| ELISA | Enzyme-linked immunosorbent assay |
| SEC-HPLC | Size exclusion chromatography-high performance liquid chromatography |
| HPLC-FLD | High performance liquid chromatography-fluorescence detection |
| CEX-HPLC | Cation exchange chromatography |

### Examples

The anti-CLDN18.2/CD3 bispecific antibody, an antibody independently developed by Innovent Biologies (Suzhou) Co., Ltd., was disclosed in PCT Application No. PCT/CN2021/121286.

In order to develop a simple and easy-to-use injection formulation formula suitable for long-term stable storage of the fully human antibody, the effect of different pH values, different stabilizers, and the content of surfactant on the quality of the antibody protein was investigated by 40 °C forced and 25 °C accelerated stability tests, and finally, a formulation formula favorable for the stability of the antibody was selected. The materials and methods used throughout the study are as follows:

### Materials and methods

### 1.1. Materials used in formulation study of present invention

| Name | Grade | Manufacturer & brand | Catalog No. |
|---|---|---|---|
| Histidine | Pharmaceutical grade | Ajinomoto, Shanghai | N/A |
| Arginine hydrochloride | Pharmaceutical grade | Ajinomoto, Shanghai | N/A |
| Sorbitol | Pharmaceutical grade | Roquette, French | H20110265 |
| Sodium acetate | Pharmaceutical grade | Merck, Germany | 1.37046.5000 |
| Edetate disodium | Pharmaceutical grade | Merck, Germany | 1.370041000 |
| Polysorbate 80 | Pharmaceutical grade | Well, Nanjing | N/A |
| Hydrochloric acid | Pharmaceutical grade | Nanjing Chemical Reagent Co., Ltd. | C0680885024 |

| | | | |
|---|---|---|---|
| Note: N/A denotes not applicable. | | | |

### 1.2. Instruments and equipment used in formulation study of present invention

| Name | Manufacturer & brand | Catalog No. |
|---|---|---|
| Ultrafiltration centrifuge tube | Millipore, USA | UFC901096 |
| Capsule filter H4 | Sartorius, Germany | 5441307H4--OO--B |
| Platinum-cured silicone tubing | Nalgene, USA | 8600-0080 |
| 2 R tubular injection vial made of neutral borosilicate glass | Schott, Suzhou | 1142144 |
| 13 mm bromobutyl rubber stopper coated with ethylene-tetrafluoroethylene copolymer film for injection | West, Germany | 7002-4373 |
| 13 mm aluminum-plastic composite cap for antibiotic vial | West, Singapore | 5413-1065 |

### 1.3. Items and methodology for formulation stability tests

The test items in the whole studies include: (1) the appearance and the presence of visible particles; (2) the protein content in the formulation determined by the ultraviolet method (UV method); (3) the turbidity of the formulation determined by the OD_{350 nm} method; (4) the purity of the antibody formulation determined by size exclusion chromatography-high performance liquid chromatography (SEC-HPLC) and expressed as the percentage of the main peak area to the sum of all peak areas; (5) the purity of the antibody formulation determined by non-reduced capillary electrophoresis-sodium dodecyl sulfate (non-reduced CE-SDS) and expressed as the percentage of the main peak area to the sum of all peak areas; (6) charge variants in the antibody formulation determined by CEX-HPLC and expressed as the percentage of the main species, acidic species, and basic species; (7) the relative binding activity of the anti-CLDN18.2/CD3 in the antibody formulation to an antigen determined by direct ELISA; and (8) the polysorbate 80 content in the antibody formulation determined by HPLC-FLD.

### Detection of visible particles

The visible particles in the sample were detected using a clarity detector (model No. YB-2, Tianda Tianfa, Tianjin) and an insoluble particle detector (model No. GWJ-8, Tianda Tianfa, Tianjin) according to the method described in the National Pharmacopoeia Committee, the Pharmacopoeia of the People's Republic of China (2015 edition, volume IV General Rules 0904 "Test for Visible Particles", Beijing, China Medical Science Press, 2015).

### Determination of protein content

The protein content of the sample was determined using an ultraviolet spectrophotometer (model No. UV-1800, Shimadzu, Japan) or a multi-channel microspectrophotometer (model No. Nanodrop 8000, Thermo, USA).

### Determination of turbidity

The turbidity of the sample was determined by measuring the absorbance at 350 nm using an ultraviolet spectrophotometer (model No. UV-1800, Shimadzu, Japan).

### Purity (SEC-HPLC)

Separation was performed on a size exclusion chromatographic column, wherein the mobile phase was a phosphate buffer (3.12 g of sodium dihydrogen phosphate dihydrate, 8.77 g of sodium chloride, and 34.84 g of arginine were dissolved in ultra-pure water, the pH was adjusted to 6.8 with hydrochloric acid, and the volume was brought to 1000 mL), the chromatographic column protective solution was 0.05% (w/v) NaN₃, the sample injection volume was 50 µL, the flow rate was 0.5 mL/min, the collection time was 30 min, the column temperature was 25 °C, and the detection wavelength was 280 nm. The test sample was diluted to 2 mg/mL with ultra-pure water for use as a sample solution. The formulation buffer was diluted in the same manner as described above to prepare a blank solution. The blank solution and the sample solution were separately injected into a liquid chromatograph in an amount of 50 µL for determination.

### Purity (non-reduced CE-SDS)

The determination was conducted by capillary gel electrophoresis. The capillary was an uncoated capillary having an inner diameter of 50 µm, a total length of 30.2 cm, and an effective length of 20.2 cm. Before electrophoresis, the capillary column was washed with 0.1 mol/L sodium hydroxide, 0.1 mol/L hydrochloric acid, ultra-pure water, and electrophoresis gel at 70 psi. The test sample was diluted to 2.0 mg/mL with an appropriate amount of ultra-pure water. 50 µL of the diluted sample was transferred into a 1.5-mL centrifuge tube, and 45 µL of sample buffer at pH 6.5 (0.32 g of citric acid monohydrate and 2.45 g of disodium phosphate dodecahydrate were dissolved in 45 mL of ultra-pure water, and the volume was brought to 50 mL to prepare a citric acid-phosphate buffer; 200 µL of the buffer was precisely measured out, 80 µL of 10% (w/v) sodium dodecyl sulfate solution was added, the volume was brought to 1 mL by adding water, and the mixture was well mixed to give the sample buffer), 1 µL of internal standard (10 kDa protein, 5 mg/mL; Beckman Coulter, Catalog No. 390953) and 5 µL of 250 mmol/L NEM solution (62 mg of N-ethylmaleimide was dissolved in 2 mL of ultra-pure water) were added. The mixture was well mixed, heated at 70±2 °C for 10±2 min, cooled to room temperature, and then transferred to a sample bottle for future use as a sample solution. The formulation buffer of the same volume as the sample was processed by the same method as above to prepare a blank solution. Conditions for sample injection: -5 kV for 20 s; separation voltage: -15 kV for 35 min. The capillary column temperature was controlled at 25 °C and the detection wavelength was 220 nm.

### Charge variants (CEX-HPLC)

The samples were detected by cation exchange chromatography (CEX-HPLC). Separation was performed using a MabPac SCX-10 strong cation exchange chromatographic column, wherein the mobile phase A was a 10 mmol/L phosphate buffer (0.51 g of NaH₂PO₄·2H₂O and 2.40 g of Na₂HPO₄·12H₂O were dissolved in 800 mL of ultra-pure water, the volume was brought to 1000 mL, and the mixture was filtered through a Φ 0.22 µm filter membrane), and the mobile phase B was a 10 mmol/L phosphate + 200 mmol/L sodium chloride buffer (0.51 g of NaH₂PO₄·2H₂O, 2.40 g of Na₂HPO₄·12H₂O, and 11.69 g of NaCl were dissolved in 800 mL of ultra-pure water, the volume was brought to 1000 mL, and the mixture was filtered through a Φ 0.22 µm filter membrane). The sample was diluted to 2.0 mg/mL with ultra-pure water as a sample solution. The formulation buffer was diluted in the same manner as described above to prepare a blank solution. The blank solution and the sample solution were separately injected into a liquid chromatograph in an amount of 50 µL for determination. The flow rate of the mobile phase was 1.0 mL/min, the collection time was 35 min, the column temperature was 35 °C, the detection wavelength was 280 nm, and the temperature of the sample tray was 10 °C. The sample solution was injected for analysis, and the contents of the main species, the acidic species, and the basic species were calculated by area normalization.

### Polysorbate 80 content (HPLC-FLD)

The samples were detected by high performance liquid chromatography-fluorescence detection (HPLC-FLD). 0.15 mol/L sodium chloride, 0.05 mol/L tris(hydroxymethyl)aminomethane (pH 8.0), 5% acetonitrile, 5.0 µmol/L N-phenyl-1-naphthylamine, and 15 ppm 23 polyoxyethylene aliphatic alcohol ether were used; isocratic elution was performed; the flow rate was 1.5 mL/min; the collection time was 3 min; the upper pressure limit was 100 bar; the sample injection volume was 10 µL; the column temperature was 30 °C; and the detection wavelengths included an excitation wavelength of 350 nm and an emission wavelength of 420 nm. The polysorbate 80 content was calculated according to the standard curve method.

### Example 1. Construction of Stably Expressing Cell Strains

### Preparation of cell strains overexpressing human CLDN18.2

According to the manufacturer's instructions, the Freedom^{®} CHO-S^{®} kit (Invitrogen, A1369601) was used to construct cell lines stably expressing human Claudin18.2 (abbreviated as CLDN18.2, the same applies hereinafter). Firstly, the full-length gene of human CLDN18.2 (UniProt ID: P56856-2) was introduced into a vector pCHO1.0 to form a plasmid, which was transfected into CHO-S cells (Invitrogen, A1369601) and HEK293 cells (Invitrogen, A14527) separately by chemical transfection and electrotransfection, and the transfected cells were subjected to two rounds of pressurized screening to obtain cell pools expressing CLDN18.2. Then, the cells highly expressing CLDN18.2 were sorted out by using a flow cytometric sorter (MoFlo XDP, Beckman Coulter), and the monoclonal cell lines CHO-hCLDN18.2 and HEK293-hCLDN18.2 stably expressing CLDN18.2 were obtained by dilution.

### Preparation of cell strain overexpressing human CLDN18.1

According to the manufacturer's instructions, the Freedom^{®} CHO-S^{®} kit (Invitrogen, A1369601) was used to construct cell lines stably expressing human Claudin18.1 (abbreviated as CLDN18.1, the same applies hereinafter). Firstly, the full-length gene of human CLDN18.1 (UniProt ID: P56856-1) was introduced into a vector pCHO1.0 (Invitrogen, A1369601) to construct a plasmid, which was transfected into CHO-S cells (Invitrogen, A1369601) by chemical transfection, and the transfected cells were subjected to two rounds of pressurized screening to obtain the cell pool expressing CLDN18.1. Then, the cells highly expressing CLDN18.1 were sorted out by using a flow cytometric sorter (MoFlo XDP, Beckman Coulter), and the monoclonal cell line CHO-hCLDN18.1 stably expressing CLDN18.1 was obtained by dilution.

### Construction of tumor cell lines overexpressing CLDN18.2

The full-length gene of human CLDN18.2 (UniProt ID: P56856-2) was introduced into a vector pWPT-GFP (Addgene, 12255) to replace the GFP sequence therein, and the construct was transfected, together with the packaging vectors psPAX2 (Addgene, 12260) and pMD2.G (Addgene, 12259) of the lentivirus, into HEK293T (ATCC, CRL-3216) cells for virus packaging. The culture supernatants cultured for 48 h and 72 h were each collected, and the lentivirus was concentrated using PEG8000. The pancreatic cancer DAN-G cells and gastric cancer KATO III cells were transfected with the concentrated virus, and then the cells expressing CLDN18.2 were sorted out by using a flow cytometric sorter (MoFlo XDP, Beckman Coulter) to obtain the tumor cell lines DAN-G-hCLDN18.2 and KATO III-hCLDN18.2 that were stably transfected with CLDN18.2.

### Example 2. Production of CLDN18.2 Monoclone

In the present invention, H2L2 fully human antibody transgenic mice (purchased from Harbour BioMed) were immunized with the cells obtained in Example 1 (CHO-hCLDN18.2) by using the hybridoma technology. Spleen cells from the mice were then harvested and electrofused with myeloma cells. Then, the supernatant was collected, hybridoma cells specifically expressing the anti-CLDN18.2 antibody were selected by a flow cytometer (FACS), and the secreted antibody did not bind to CLDN18.1. The test cells (HEK293-hCLDN18.2) obtained in Example 1 were counted, diluted to 1 × 10⁶ cells/mL, and then added to a U-bottom 96-well plate at 100 µL/well. The cells were centrifuged at 500 g for 5 min, and the cell medium was removed. The hybridoma culture supernatant in a 96-well plate was added to the U-bottom plate at 100 µL/well, the cells were resuspended, and the suspension was left to stand on ice for 30 min. The suspension was centrifuged at 500 g for 5 min, the supernatant was removed, and the cells were washed once with PBS. 100 µL of FITC-labeled anti-mouse Fab secondary antibody (1:500 dilution in PBS) was added to each well, and 100 µL of FITC-labeled anti-human Fab secondary antibody was added to the positive control antibody. The resulting mixture was incubated on ice in the dark for 30 min. The mixture was centrifuged at 500 g for 5 min, the supernatant was removed, and the cells were washed once with PBS. The cells were resuspended in 50 µL of 1× PBS, and the suspension was loaded for FACS assay. The positive clones were rescreened for CHO-hCLDN18.1 in the same manner as described above. After two rounds of screening, the fully human antibody clone HB37A6 was obtained.

### Example 3. Preparation of Recombinant CLDN18.2 Monoclonal Antibody

The anti-CLDN18.2 monoclonal antibody HB37A6 (see CN202010570517.X) and the control antibody zolbetuximab (abbreviated as Zmab, sequence derived from INN117) were expressed as full-length monoclonal antibodies in HEK293 cells (Invitrogen, A14527).

Expression vectors were first constructed. The heavy chain variable region and light chain variable region of HB37A6 and those of the control antibody (see sequence information) were separately placed at the N-terminus of the human IgG1 heavy chain constant region (SEQ ID NO: 5) and the light chain kappa constant region (SEQ ID NO: 10). Then, the obtained sequences were introduced into pcDNA3.1 expression vectors with N-terminal signal peptide to obtain the light and heavy chain expression vectors. The obtained light and heavy chain expression vectors were co-transfected into HEK293 cells by PEI (Polysciences Inc, 23966), and the medium supernatant was collected after 7 days of culturing. The supernatant was purified by a Protein A column (Hitrap Mabselect Sure, GE 11-0034-95), followed by ultrafiltration and buffer-exchange into PBS (Gibco, 70011-044). The concentration was determined by the A280 method and the purity was determined by the SEC-HPLC method to obtain an antibody solution with a purity of greater than 95%, thus obtaining the recombinant CLDN18.2 monoclonal antibody HB37A6.

The specific transfection and purification procedures were as follows:
Expi293 cells (Invitrogen, A14527) were passaged according to a desired transfection volume. The cell density was adjusted to 1.5 × 10⁶ cells/mL the day before transfection. The cell density on the day of transfection was approximately 3 × 10⁶ cells/mL. The Opti-MEM culture medium (Gibco, 31985-070) at a volume 1/10 of the final volume was used as the transfection buffer, the plasmid was added at 1.0 µg/mL transfected cells, and the mixture was well mixed. An appropriate amount of polyethylenimine (PEI) (Polysciences, 23966) was added to the plasmid (the ratio of plasmid to PEI was 1:3 in the 293F cells), and the mixture was well mixed and incubated at room temperature for 20 min, thus obtaining a DNA/PEI mixture. The DNA/PEI mixture was added slowly to the cells while gently shaking the shake flask, and then the resulting mixture was cultured in an incubator at 36.5 °C and 8% CO₂; after culturing for seven days, a cell suspension was obtained, and the cell supernatant was collected for purification.

The Protein A column (Hitrap Mabselect Sure, GE, 11-0034-95) used for purification was treated with 0.1 M NaOH for 2 h, and the glass bottles or the like were washed with distilled water and then dried at 180 °C for 4 h. The collected cell suspension was centrifuged at 4500 rpm for 30 min before purification, and the supernatant was filtered using a 0.22 µm filter. The Protein A column was equilibrated with 10 column volumes of binding buffer (20 mM sodium phosphate, 150 mM NaCl, pH 7.0). The filtered supernatant was loaded to the purification column, which was then equilibrated with 10 column volumes of binding buffer. 5 mL of elution buffer (0.1 M citric acid + sodium citrate, pH 3.5) was added. The eluate was collected, and 80 µL of 2 M Tris-HCl was added per mL of eluate. The collected antibody was buffer-exchanged into PBS (Gibco, 70011-044) by ultrafiltration concentration, and the concentration was measured.

### Example 4. Preparation of Anti-CD3 Monoclonal Antibodies

The murine CD3 antibody sp34 (U.S. Pat. No. 8,236,308; J. Immunol. Methods., 1994, 178: 195) and the humanized and affinity-optimized anti-CD3 monoclonal antibodies of sp34, HzSP34.24, HzSP34.87, and HzSP34.97, were expressed in HEK293 cells (Invitrogen, A14527) as full-length monoclonal antibodies.

The heavy chain variable regions heavy chain variable regions and light chain variable regions of HzSP34.24, HzSP34.87, HzSP34.97, and sp34 (see sequence information) were placed at the N-terminus of the IgG1 heavy chain constant region (SEQ ID NO: 23) and the light chain lambda constant region (SEQ ID NO: 28). Then, the obtained sequences were introduced into pcDNA3.1 expression vectors with N-terminal signal peptide to obtain the light and heavy chain expression vectors. The obtained light and heavy chain expression vectors were co-transfected into HEK293 cells by PEI (Polysciences Inc, 23966), and the medium supernatant was collected after 7 days of culturing. The supernatant was purified by a Protein A column (Hitrap Mabselect Sure, GE 11-0034-95), followed by ultrafiltration and buffer-exchange into PBS (Gibco, 70011-044). The concentration was determined by the A280 method and the purity was determined by the SEC-HPLC method to obtain an antibody solution with a purity of greater than 95%, thus obtaining the recombinant anti-CD3 monoclonal antibodies HzSP34.24, HzSP34.87, and HzSP34.97, as well as the murine CD3 antibody sp34. The specific transfection and purification procedures are shown in Example 3.

### Example 5: Construction and Preparation of Bispecific Antibodies

According to the combinations in Table 2, the sequences of the antigen-binding regions of the anti-CLDN18.2 monoclonal antibody HB37A6 and 3 different anti-human CD3 monoclonal antibodies HzSP34.24, HzSP34.87, and HzSP34.97 were separately used to construct bispecific antibodies, 030, 032, and 033, targeting CLDN18.2×CD3 in the "1+1" form. The schematic structural diagram of the antibody is shown in FIG. 1. Specifically, the heavy chain variable region sequence of the antigen-binding domain specifically targeting CLDN18.2 was SEQ ID NO: 4, and the light chain variable region sequence was SEQ ID NO: 9; the heavy chain variable region sequences of the antigen-binding domains specifically targeting CD3 were SEQ ID NO: 22, SEQ ID NO: 30, and SEQ ID NO: 32, and the light chain variable region sequence was SEQ ID NO: 27. The IgG1 LALA sequence of the knob-into-hole structure was selected for the Fc fragment of the antibody (A. Margaret Merchant et al., Nature Biotechnology, 1998). Thus, the heavy chain of the CLDN18.2 moiety of the bispecific antibody was set forth in SEQ ID NO: 37, and the light chain was set forth in SEQ ID NO: 38. The heavy chains of the CD3 moiety of the bispecific antibody were set forth in SEQ ID NOs: 39, 41, and 42, respectively, and the light chain was set forth in SEQ ID NO: 40.

The plasmid construction process for the bispecific antibody was as follows: the CLDN18.2 heavy chain sequence (SEQ ID NO: 37), the CLDN18.2 light chain sequence (SEQ ID NO: 38), the CD3 heavy chain sequence (SEQ ID NOs: 39, 41, and 42), and the CD3 light chain sequence (SEQ ID NO: 40) were separately inserted into a vector pcDNA3.1 (Invitrogen, V790-20) to obtain heavy and light chain plasmids of the CLDN18.2 moiety and heavy and light chain plasmids of the CD3 moiety. The heavy and light chain plasmids of the CLDN18.2 moiety and the heavy and light chain plasmids of the CD3 moiety were then transiently transfected into Expi293 cells (Invitrogen, A14527) using PEI (Polysciences, 23966) to express 3 antibody molecules of the CLDN18.2 moiety and CD3 moiety. After 7 days, the cell fermentation broths were obtained, filtered, clarified, and captured using a Protein A column of Hitrap Mabselect Sure (GE Healthcare, 11-0034-95) to obtain antibodies of the CLDN18.2 and CD3 moieties. After the concentration was determined by the A280 method, the antibodies of both moieties were mixed in a molar ratio of 1:1. A proper amount of the reducing agent GSH was added, and the mixture was reacted at room temperature overnight. Then, the reducing agent was removed by ultrafiltration, and the reaction was terminated. Thereafter, fine purification was performed using MonoS cation exchange chromatography (GE Healthcare, 17-5168-01), with a 20 mM sodium phosphate buffer (pH 6.6) as solution A and a 20 mM sodium phosphate buffer (pH 6.6) containing 1 M sodium chloride as solution B. The elution gradient was 0-50% (30 column volumes). The eluted protein solution was ultrafiltered and buffer exchanged into PBS (Gibco, 70011-044). The molecular weight was determined by mass spectrometry, and the purity was determined by SEC-HPLC. The obtained bispecific antibodies 030, 032 and 033 were used in the following examples.

**Table 2. List of CD3/CLDN18.2 bispecific antibodies**

| | Anti-CLDN18.2 moiety | Anti-CD3 moiety |
|---|---|---|
| 030 | HB37A6 | HzSP34.24 |
| 032 | HB37A6 | HzSP34.87 |
| 033 | HB37A6 | HzSP34.97 |

### Example 6. Determination of affinities of Bispecific Antibodies

The equilibrium dissociation constant (KD) for binding of the bispecific antibodies of the present invention to human CD3 protein was determined by bio-layer interferometry (BLI). A BLI affinity assay was conducted according to the existing method (Estep, P et al., High throughput solution Based measurement of antibody-antigen affinity and epitope binning, MAbs, 2013.5(2): p. 270-8).

Half an hour before the experiment, an appropriate number of AHC sensors (18-5060, Fortebio) were taken according to the number of samples and soaked in an SD buffer (1× PBS, BSA 0.1%, Tween-20 0.05%). 100 µL of the SD buffer, bispecific antibodies, and the human CD3 protein (CT026H0323H, Beijing Sino Biological) were added to a 96-well black polystyrene half-area microplate (Greiner, 675076). Detection was conducted using Fortebio Octet Red96, and the sensors were arranged according to the positions of the samples. The instrument settings were as follows: the operation procedures were Baseline, Loading -1 nm, Baseline, Association, and Dissociation; the run time of each procedure was dependent on the rates of association and dissociation of samples; the rotation speed was 1000 rpm; and the temperature was 30 °C. The KD values were analyzed using ForteBio Octet analysis software.

The affinities of the bispecific antibodies are shown in Table 3. Among them, the affinity of the CD3 moiety of the molecule 030 was the highest, which was 7.4 nM. The affinities of the CD3 moieties of the molecules 032 and 033 were reduced sequentially, which were 89 nM and 440 nM, respectively.

**Table 3. Affinities of CD3 moieties of bispecific antibodies**

| | Ka (1/Ms) | Kd (1/s) | KD (M) |
|---|---|---|---|
| 030 | 6.927E+5 | 0.005164 | 7.455E-9 |
| 032 | 8.066E+5 | 0.07183 | 8.906E-8 |
| 033 | 3.788E+5 | 0.1689 | 4.459E-7 |

The equilibrium dissociation constant (KD) for binding to human CLDN18.2 was determined by surface plasmon resonance (SPR). According to the manufacturer's instructions, the antigen human Claudin18.2 (GenScrip, P50251802) was coupled to the surface of a CM5 chip (GE Healthcare, 29-1496-03) using the amino coupling kit (GE Healthcare, BR-1006-33), and the remaining activation sites were blocked by injection of 1 M ethanolamine after coupling. According to the manufacturer's instructions, the binding and dissociation between the antigen on the chip surface and each bispecific antibody in the mobile phase were detected by Biacore (GE Healthcare, T200) to obtain affinity and kinetic constants. The antibodies (0-100 nM, 2-fold dilution) obtained by gradient dilution flowed over the chip surface in an order from low concentration to high concentration, with the binding time of 180 s and the dissociation time of 600 s. Finally, the chip was regenerated using 10 mM Glycine pH 1.5 (GE Healthcare, BR-1003-54). Kinetic analysis of the data results was performed using Biacore T200 analysis software with a 1:1 binding model. The results are shown in Table 4. The bispecific antibodies 030,032 and 033 adopted the same clone HB37A6 in the CLDN18.2 moieties, and the affinities of the CLDN18.2 moieties were consistent, with a very strong affinity of 0.57 nM.

**Table 4. Affinities of CLDN18.2 moieties of bispecific antibodies**

| | Ka (1/Ms) | Kd (1/s) | K_{D}(M) |
|---|---|---|---|
| 030 | 3.47E+05 | 2.00E-04 | 5.76E-10 |
| 032 | | | |
| 033 | | | |

### Example 7. In Vitro T Cell Killing Experiment

The bispecific antibodies 030, 032 and 033 obtained in Example 5 were subjected to *in vitro* T cell killing experiments. The human peripheral blood mononuclear cells (PBMCs, Allcells or Saily) were resuspended in complete medium RPMI-1640 (Hyclone, SH30809.01) + 10% fetal bovine serum (FBS, Hyclone, SH30084.03), and the PBMCs were adjusted to 2 × 10⁶ cells/mL.

NUGC-4, DAN-G tumor target cells overexpressing Claudin18.2 DAN-G-hCLDN18.2, and non-target cells L363 (DSMZ, ACC49) were labeled with Far-Red (Invitrogen) for 10 min, washed twice, and then resuspended in the complete medium with the cell concentration adjusted to 2 × 10⁵ cells/mL.

The PBMCs were mixed with each of the bispecific antibodies 030, 032 and 033 (wherein 030 and 032 were used at an initial concentration of 1 nM, 033 was used at an initial concentration of 400 nM, and all antibodies were subjected to 5-fold dilution, for a total of 10 concentration points). The mixture was incubated at 37 °C for 30 min, and then 50 µL of tumor target cells (1 × 10⁴) were added to 50 µL of PBMC effector cells in an effector-to-target ratio of 10:1. The mixture was incubated at 37 °C for 24 h and centrifuged; the cells were resuspended in propidium iodide (PI, Invitrogen) at a final concentration of 10 µg/mL; and the Far-Red and PI double positive cells were detected by a flow cytometer (BD, FACSCELESTA). The killing ratio of tumor target cells was calculated by FACSDiva software (BD, Celestsa).

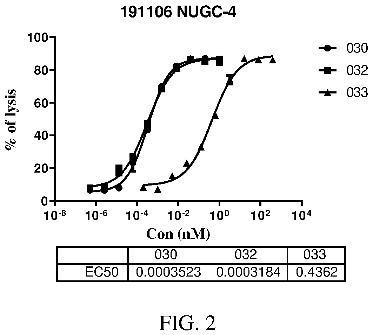

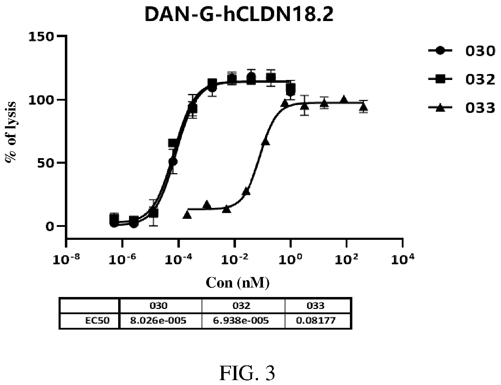

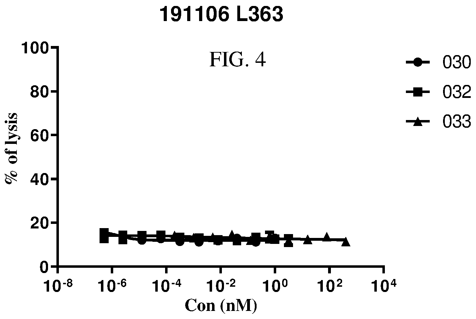

From the results shown in FIG. 2, it is shown that 030 and 032 molecules had very strong killing activity against gastric cancer cells NUGC-4, and the EC50 values were both less than 1 pM. From the results shown in FIG. 3, it is shown that the EC50 values of the two molecules were even less than 0.1 pM in DAN-G-hCLDN18.2, a pancreatic cancer cell with high expression of CLDN18.2. The molecule 033 had weaker killing activity against the two tumor cell lines, which was about 1000 times lower than those of 030 and 032, but it still reached the maximal killing (nearly 100% lysed cells). However, in the case of negative expression of CLDN18.2, the molecules 030,032 and 033 all have no non-specific killing (FIG. 4). This indicates that the molecules 030,032 and 033 all exhibit specific killing against tumor cells dependent on CLDN18.2 expression. Furthermore, the killing effects of the bispecific antibodies of the present invention are related to the abundance of CLDN18.2 on the cell surface. Within a certain range of expression abundance, the higher the expression level of CLDN18.2 on the cell surface, the better the killing effect.

### Example 8. In Vivo Efficacy Experiment - Gastric Cancer Model

This experiment studied the anti-tumor effects of the bispecific antibodies in NUGC-4 tumor-bearing NOG female mice. Forty-nine NOG female mice (Beijing Vital River Laboratory Animal Technology Co., Ltd.) were selected. PBMC cells (Allcells) were thawed and centrifuged. The PBMC cells were dispersed with PBS (1×) to obtain a cell suspension with a cell density of 2 × 10⁷ cells/mL. 200 µL of the cell suspension was taken, and the PBMC cells were injected into each of the mice via the orbital vein at 4 × 10⁶ cells/mouse.

The NUGC-4 cells were thawed and subcultured conventionally for subsequent *in vivo* experiments. The cells were collected by centrifugation, and the NUGC-4 cells were dispersed with PBS (1×) to obtain a suspension with a cell density of 6 × 10⁷ cells/mL. The suspension was mixed with matrigel at 1:1 to prepare a cell suspension with a cell concentration of 3 × 10⁷ cells/mL. On day 3, 0.2 mL of the cell suspension was subcutaneously inoculated into the right abdominal region of each of the NOG humanized mice to establish an NUCG-4 tumor-bearing mouse model.

The maximum length of minor axis and the maximum length of major axis of the tumors of the mice were measured by using a vernier caliper 7 days after cell inoculation, and the tumor volume was calculated. The mice with a tumor volume within 53.35 mm³ to 168.07 mm³ were selected and divided into groups in a serpentine manner according to the tumor volume of the mice (6 mice in each group). The bispecific antibodies 030, 032 and 033 of the present invention and a negative control h-IgG (Equitech-Bio, batch No. 160308-02) were injected intravenously into each mouse at administration doses of 0.3 mg/kg and 1 mg/kg once a week for a total of 4 times. The tumor volume of the mice was measured twice a week. The tumor growth inhibition (TGI%) was calculated simultaneously, and the calculation formula is as follows: TGI% = 100% × (tumor volume of the control group - tumor volume of the treatment group)/(tumor volume of the control group - tumor volume of the control group before administration).

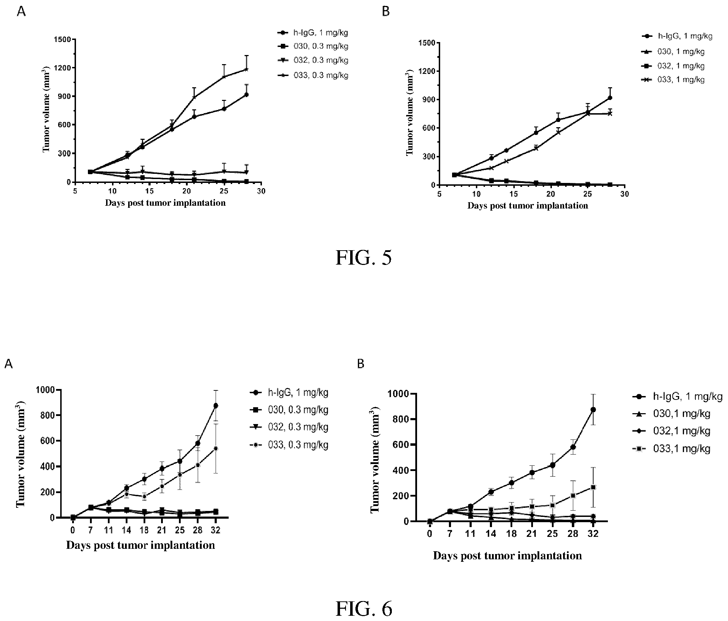

As shown in FIG. 5, in the human gastric cancer NUCG-4 tumor-bearing mouse model, 030 and 032 were capable of achieving TGI 100% at a low dose of 0.3 mg/kg and even achieving 50% CR (complete remission) at a high dose of 1 mg/kg (complete regression of the tumor was achieved in 3 out of 6 mice). However, the molecule 033 had little efficacy at the low dose, and had a TGI of up to 20% at 1 mg/kg. Throughout the experiment, the mice in the experimental groups and the control group did not lose weight.

### Example 9. In Vivo Efficacy Experiment - Pancreatic Cancer Model

This experiment studied the anti-tumor effects of the bispecific antibodies in DAN-G-Claudin18.2 tumor-bearing NOG female mice. Forty-nine NOG mice (Beijing Vital River Laboratory Animal Technology Co., Ltd.) were injected with PBMC cells via the orbital vein at 4 × 10⁶ cells/mouse in an inoculation volume of 200 µL/mouse (as shown in Example 18). This day was recorded as day 0.

The human pancreatic cancer cells DAN-G-CLDN18.2 constructed in Example 1 were subcultured conventionally for subsequent *in vivo* experiments. The cells were collected by centrifugation, and the DAN-G-CLDN18.2 cells were dispersed with PBS (1×) to obtain a suspension with a cell density of 10 × 10⁶ cells/mL. The cell suspension was mixed with matrigel at 1:1 to prepare a cell suspension with a cell concentration of 5 × 10⁶ cells/mL. On day 0, 0.2 mL of the cell suspension was subcutaneously inoculated into the right abdominal region of each of the NOD-SCID mice to establish a DNA-G pancreatic cancer humanization model with the overexpression of CLDN18.2.

The maximum length of minor axis and the maximum length of major axis of the tumors of the mice were measured by using a vernier caliper 7 days after tumor cell inoculation, and the tumor volume was calculated. The mice were divided into groups in a serpentine manner according to the tumor volume of the mice, that is, the mice with a tumor volume in the range of 46.42 mm³ to 120.64 mm³ were divided into groups in the serpentine manner according to the tumor volume (6 mice in each group).

The bispecific antibodies 030, 032 and 033 of the present invention and the negative control h-IgG (Equitech-Bio, batch No. 160308-02) were injected intravenously into each mouse at intraperitoneal administration doses of 0.3 mg/kg and 1 mg/kg once a week for a total of 4 times. The tumor volume of the mice was measured twice a week. The tumor growth inhibition (TGI%) was calculated simultaneously, and the calculation formula is as follows: TGI% = 100% × (tumor volume of the control group - tumor volume of the treatment group)/(tumor volume of the control group - tumor volume of the control group before administration). As shown in FIG. 6, in the DNA-G pancreatic cancer humanization model with the overexpression of CLDN18.2, molecules 030 and 032 were capable of achieving TGI 100% at both doses. The molecule 033 also achieved TGI 42% (0.3 mg/kg) and TGI 76% (1 mg/kg), which may be related to the high expression level of CLDN18.2 in DAN-G-CLDN18.2 pancreatic cancer cells. Throughout the experiment, the mice in the experimental groups and the control group did not lose weight.

### Example 10. PK Experiment in Mice

In this study, 030, 032 and 033 were injected into female Balb/C mice (Vital River) at 10 mg/kg via the tail vein to study their pharmacokinetic properties in the mice. After administration to the mice, blood was taken from the eyeball at time points of 0.086 h, 0.5 h, 2 h, 6 h, 24 h, 48 h, 4 days, 7 days, 14 days, and 21 days and centrifuged at 3000 rpm at 4 °C for 10 min, and serum was collected. The antibody content in the serum was determined by ELISA, and the half-lives of 030, 032 and 033 in the mice were calculated.

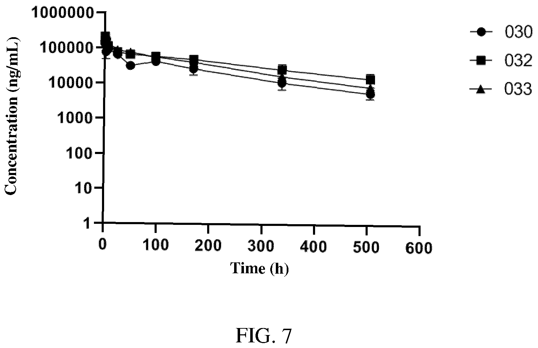

The experimental results are shown in FIG. 7. The half-lives of 030, 032 and 033 in the mice all reached similar PK to the normal monoclonal antibody. It was further demonstrated that the bispecific antibodies constructed in the present invention did not affect the half-lives of the antibodies.

### Example 11. pH Screening Experiment

### 1. Experimental procedures

A buffer containing 1.55 mg/mL histidine and 50.00 mg/mL sorbitol was prepared, and the pH was adjusted to 5.0, 5.5, 6.0, 6.5, and 7.0 with hydrochloric acid; the CLDN18.2/CD3 protein was exchanged into the buffers with different pH values by ultrafiltration, and the protein content was adjusted to about 1 mg/mL; polysorbate 80 was added at a final concentration of 0.2 mg/mL; and the resulting mixtures were filtered and aliquoted into vials, followed by stoppering and capping. The stability of the samples described above was investigated at 40±2 °C, and the specific scheme is shown in the Table.

**Table 5. Experimental scheme**

| Experimental condition | Sampling ti me points | | | | | Test items |
|---|---|---|---|---|---|---|
| | Week 0 | Week 1 | Wee k 2 | | Week 4 | |
| 40°C±2°C | √ | | √ | | √ | Appearance, visible particles, protein content, pH, purity, charge variants, and biological activity |
| | | | | | | |
| | | | | | | |
| | | | | | | |

| | | | | | | |
|---|---|---|---|---|---|---|
| Note: (1) √ represents that sampling is performed at this time point (2) After sampling at the time points described above, the samples were first cryopreserved at -70 °C, and then thawed before detection as required. | | | | | | |

### 2. Criteria

According to the knowledge of the product and the precision of the instrument and the method, criteria for determining the absence of quality change in sample measurements as compared to initial values were set.

**Table 6. Cr riteria for determining abs ence of quality change**

| Test items | | | | Criteria for determi ining absence of change | | |
|---|---|---|---|---|---|---|
| Appearance ( visual inspec tion) | | | | Clear to slightly opalescent, colorless to yellowish liquid, free of particles | | |
| Visible particles (test for visible particles) | | | | No visible particles | | |
| Protein conten t (UV meth od) | | | | Change rate ≤ 10% | | |
| pH value (met thod for dete rmining pH) | | | | Change value ≤ 0.3 | | |
| Purity (SEC- HPLC) | | | | Change in main pe ak purity ≤ 1% | | |
| Purity (non-re duced CE-SDS) | | | | Change in main pe ak purity ≤ 2% | | |
| Charge variants (CEX-HP LC) | | | | Changes in main species, acidic species, and basic species ≤ 2% | | |
| Polysorbate 80 content (H PLC-FLD)* | | | | 0.1 mg/ml-0.3 mg/ ml | | |
| Biological acti vity | | | | 70-130% | | |

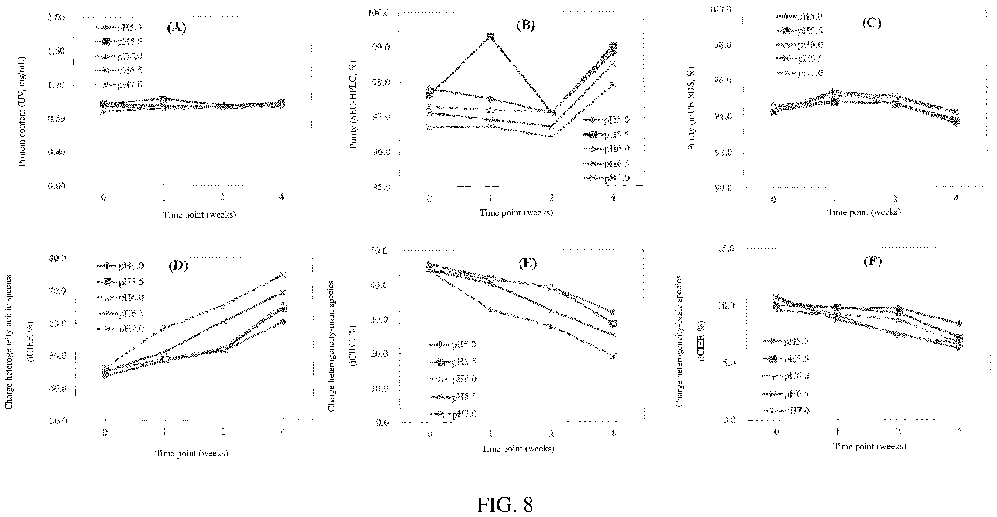

The results (as shown in FIG. 8) show that the samples were all qualified in appearance and visible particles under various pH conditions after storage at 40±2 °C for 4 weeks; the protein content and pH were not significantly changed. The purity (nrCE-SDS) of the samples under various pH conditions was not significantly changed, and the purity (SEC-HPLC) of other samples was increased except that the sample at a pH of 5.0 had no significant change. The charge variants-acidic species of each sample were increased significantly, and the acidic species were increased faster with the increase in the pH value; the charge variants-main species of each sample were decreased significantly, and the main species were decreased faster with the increase in the pH value; the charge variants-basic species of other samples were decreased significantly except that the sample at a pH of 5.0 had no significant change; the biological activity of the anti-Claudin 18.2 met the criteria; the biological activity of the anti-CD3 moiety failed to meet the criteria for determination under other conditions, except that the sample at a pH of 5.0 sample met the criteria.

Combining the results of the charge variants and the biological activity of the anti-CD3 moiety, and considering the fluctuation of pH during actual production, the final pH was determined to be 5.2 for the formula determination experiment.

### Example 12. Formula Determination Experiment

In this example, the effects of buffer systems (histidine system and sodium acetate system) and auxiliary materials (sorbitol, arginine hydrochloride, and edetate disodium) on the stability of the bispecific antibody protein were mainly investigated, and the detailed formula information is shown in Table 7.

Buffers of the formulas were prepared according to Table 7, and the bispecific protein was exchanged into each of the formula solutions by ultrafiltration. The protein content in each formula solution was adjusted to 1 mg/mL after the exchange, and then polysorbate 80 was added at a final concentration of 0.2 mg/mL. The solutions were filtered and aliquoted into vials, followed by stoppering and capping. The stability of the samples described above was investigated at 40±2 °C.

**Table 7. Formula information**

| No. | Formula inf ormation | | | | |
|---|---|---|---|---|---|
| Formula 1 | 1.55 mg/mL histidine, 50. 00 mg/mL s orbitol, and 0. 2 mg/mL polysorbate 80, pH 5.2 | | | | |
| Formula 2 | 1.55 mg/mL polysorbate histidine, 50. 80, pH 5.2 00 mg/mL s orbitol, 0.01 mg/mL edetate disodium, and 0.2 mg/mL | | | | |
| Formula 3 | 1.55 mg/mL histidine, 37. 92 mg/mL a rginine hydro chloride, and 0.2 mg/mL polysorbate 80, pH 5.2 | | | | |
| Formula 4 | 1.55 mg/mL polysorbate histidine, 25. 80, pH 5.2 00 mg/mL s orbitol, 16.85 mg/mL arginine hydrochloride, and 0.2 mg/mL | | | | |
| Formula 5 | 0.82 mg/mL sodium aceta te, 50.00 mg /mL sorbitol, and 0.2 mg/mL polysorbate 80, pH 5.2 | | | | |
| Note: the pH was adjusted with hydroch loric acid | | | | | |

| Ta ble 8. Stabili ty investigati ion scheme | | | | | |
|---|---|---|---|---|---|
| | | | | | |

| Experimental condition | Sampling time points | | | | Test items |
|---|---|---|---|---|---|
| | Week 0 | Week 1 | Week 2 | Week 4 | |
| 40 °C±2 °C | √ | √ | √ | √ | Appearance, visible particles, protein content, pH, purity, charge variants, polysorbate 80 content, and biological activity |

| | | | | | |
|---|---|---|---|---|---|
| Note: (1) √ represents that sampling is performed at this time point. (2) After sampling at the time points described above, the samples were first cryopreserved in a freezer at -70 °C for later detection, and then thawed before detection as required. | | | | | |

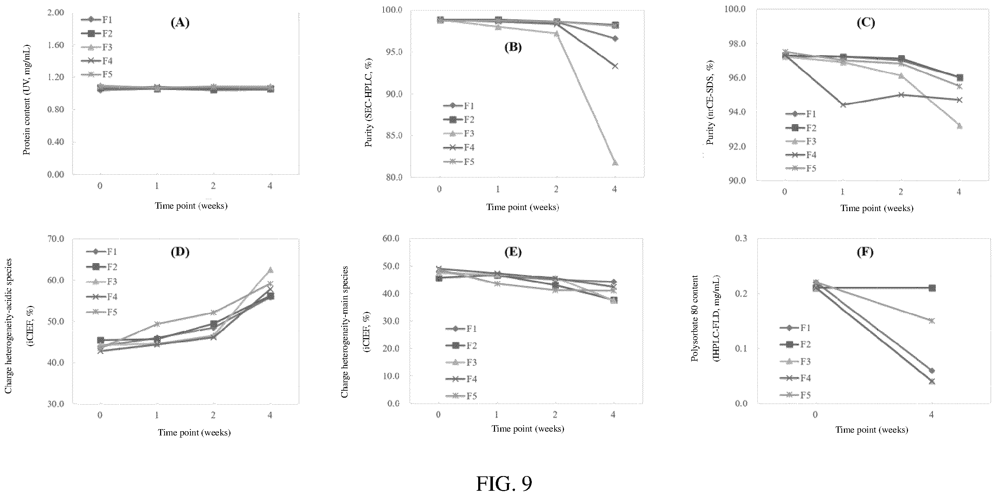

The criteria for determination are shown in Table 6. The results (as shown in FIG. 9) show that the samples of all formulas were qualified in appearance and visible particles after storage at 40±2 °C for 4 weeks; the protein content and pH values were not significantly changed. Except that the purity (SEC-HPLC) of the formula 2 and formula 5 samples was not changed significantly, the changes of other formula samples failed to meet the criteria for determination. The purity (nrCE-SDS) of the formula 3 and formula 4 samples failed to meet the criteria for determination; the formula 1 and formula 2 samples had the same downward trend, which was slower than that of the formula 5 sample. The changes in charge variants of the samples of all formulas showed a significant increase in acidic species and a significant decrease in main species. The biological activities of the anti-Claudin18.2 moieties of all formulas met the criteria; the biological activities of the anti-CD3 moieties of formula 2 and formula 5 both met the criteria, and other formulas failed to meet the criteria for determination. Except that the polysorbate 80 content of formula 2 was not changed, and other formulas all showed decreases to different degrees.

Combining the results of Example 11 and Example 12, formula 2 was selected as the final formulation formula for the anti-CLDN18.2/CD3 bispecific antibody. To avoid the adjustment of pH with hydrochloric acid during production, the buffer system was adjusted to histidine and histidine hydrochloride, i.e., the formulation formula for the anti-CLDN18.2/CD3 bispecific antibody was: 1.0 mg/mL recombinant anti-cluster of differentiation 3 (CD3) and anti-tight junction protein 18.2 (Claudin18.2) bispecific antibody, 0.18 mg/mL histidine, 1.84 mg/mL histidine hydrochloride, 50.00 mg/mL sorbitol, 0.01 mg/mL edetate disodium, and 0.2 mg/mL polysorbate 80, with a pH of 5.2.

The exemplary embodiments of the present invention have been described above. It should be understood by those skilled in the art that these contents are merely exemplary, and various other replacements, adaptations, and modifications can be made within the scope of the present invention. Therefore, the present invention is not limited to the specific embodiments listed herein.

### Sequence information

| SEQ ID NO | Name of antibody | HB37A6 |
|---|---|---|
| 1 | HCDR1 | GFTFSSYVMS |
| 2 | HCDR2 | TISHSGGSTYYADSVKG |
| 3 | HCDR3 | DAPYYDILTGYRY |
| 4 | Heavy chain variable region (VH) | EVQLLDSGGGLVQPGGSLRLSCAASGFTFSSYVMSWVRQAPGKGLN WVSTISHSGGSTYYADSVKGRFTISRDNSKNTLYLQMNSLRAEDTAV YYCAIDAPYYDILTGYRYWGQGTLVTVSS |
| 5 | Heavy chain constant region (HC) | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTCV VVDVSHEDPEVKFNWYVDGVEVHNAKTKPREEQYNSTYRVVSVLT VLHQDWLNGKEYKCKVSNKALPAPIEKTISKAKGQPREPQVYTLPPS |
| | | RDELTKNQVSLTCLVKGFYPSDIAVEWESNGQPENNYKTTPPVLDSD GSFFLYSKLTVDKSRWQQGNVFSCSVMHEALHNHYTQKSLSLSPGK |
| 6 | LCDR1 | RASQSISSWLA |
| 7 | LCDR2 | KASSLES |
| 8 | LCDR3 | QQYNSYSYT |
| 9 | Light chain variable region (VL) | DIQMTQSPSTLSASVGDRVTITCRASQSISSWLAWYQQKPGKAPKLLI YKASSLESGVPSRFSGSGSGTEFTLTISSLQPDDFATYYCQQYNSYSYT FGQGTKLEIK |
| 10 | Light chain constant region (LC) | |

| SEQ ID NO | Name of antibody | Positive control antibody Zmab |
|---|---|---|
| 11 | HCDR1 | GYTFTSYWIN |
| 12 | HCDR2 | NIYPSDSYTNYNQKFK |
| 13 | HCDR3 | SWRGNSFDY |
| 14 | VH | QVQLQQPGAELVRPGASVKLSCKASGYTFTSYWINWVKQRPGQGLE WIGNIYPSDSYTNYNQKFKDKATLTVDKSSSTAYMQLSSPTSEDSAVY YCTRSWRGNSFDYWGQGTTLTVSS |
| 5 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KAEPKSCDKTHTCPPCPAPELLGGPSVFLFPPKPKDTLMISRTPEVTCV VVDVSHEDPEVKFNWYVDGVEVHNAKTKPREEQYNSTYRVVSVLT VLHQDWLNGKEYKCKVSNKALPAPIEKTISKAKGQPREPQVYTLPPS RDELTKNQVSLTCLVKGFYPSDIAVEWESNGQPENNYKTTPPVLDSD GSFFLYSKLTVDKSRWQQGNVFSCSVMHEALHNHYTQKSLSLSPGK |
| 15 | LCDR1 | KSSQSLLNSGNQKNYLT |
| 16 | LCDR2 | WASTRES |
| 17 | LCDR3 | QNDYSYPFT |
| 18 | VL | DIVMTQSPSSLTVTAGEKVTMSCKSSQSLLNSGNQKNYLTWYQQKP GQPPKLLIYWASTRESGVPDRFTGSGSGTDFTLTISSVQAEDLAVYYC QNDYSYPFTFGSGTKLEIK |
| 10 | LC | |

| SEQ ID NO | Name of antibody | HzSP3424 |
|---|---|---|
| 19 | HCDR1 | GFTFNTYAMN |
| 20 | HCDR2 | RIRSKYNNYATYYADSVKD |
| 21 | HCDR3 | HGNFGQSYVSWFAY |
| 22 | VH | EVQLVESGGGLVQPGGSLKLSCAASGFTFNTYAMNWVRQASGKGLE WVGRIRSKYNNYATYYADSVKDRFTISRDDSKSTLYLQMNSLKTEDT AVYYCARHGNFGQSYVSWFAYWGQGTTVTVSS |
| 23 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 24 | LCDR1 | RSSTGAVTTSNYAN |
| 25 | LCDR2 | GTNKRAP |
| 26 | LCDR3 | ALWYSNLWV |
| 27 | VL | QAVVTQEPSLTVSPGGTVTLTCRSSTGAVTTSNYANWVQQKPGQAPR GLIGGTNKRAPGVPARFSGSLLGDKAALTLLGAQPEDEAEYYCALW YSNLWVFGQGTKLTVL |
| 28 | LC | GQPKAAPSVTLFPPSSEELQANKATLVCLISDFYPGAVTVAWKADSSP VKAGVETTTPSKQSNNKYAASSYLSLTPEQWKSHRSYSCQVTHEGST VEKTVAPTECS |

| SEQ ID NO | Name of antibody | HzSP34.87 |
|---|---|---|
| 19 | HCDR1 | GFTFNTYAMN |
| 20 | HCDR2 | RIRSKYNNYATYYADSVKD |
| 29 | HCDR3 | HYNFGQSYVSWFAY |
| 30 | VH | EVQLVESGGGLVQPGGSLKLSCAASGFTFNTYAMNWVRQASGKGLE WVGRIRSKYNNYATYYADSVKDRFTISRDDSKSTLYLQMNSLKTEDT AVYYCARHYNFGQSYVSWFAYWGQGTTVTVSS |
| 23 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 24 | LCDR1 | RSSTGAVTTSNYAN |
| 25 | LCDR2 | GTNKRAP |
| 26 | LCDR3 | ALWYSNLWV |
| 27 | VL | QAVVTQEPSLTVSPGGTVTLTCRSSTGAVTTSNYANWVQQKPGQAPR GLIGGTNKRAPGVPARFSGSLLGDKAALTLLGAQPEDEAEYYCALW YSNLWVFGQGTKLTVL |
| 28 | LC | GQPKAAPSVTLFPPSSEELQANKATLVCLISDFYPGAVTVAWKADSSP VKAGVETTTPSKQSNNKYAASSYLSLTPEQWKSHRSYSCQVTHEGST VEKTVAPTECS |

| SEQ ID NO | Name of antibody I | HzSP34.97 |
|---|---|---|
| 19 | HCDR1 | GFTFNTYAMN |
| 31 | HCDR2 | RIRSKAGGYATYYADSVKD |
| 21 | HCDR3 | HGNFGQSYVSWFAY |
| 32 | VH | EVQLVESGGGLVQPGGSLKLSCAASGFTFNTYAMNWVRQASGKGLE WVGRIRSKAGGYATYYADSVKDRFTISRDDSKSTLYLQMNSLKTEDT AVYYCARHGNFGQSYVSWFAYWGQGTTVTVSS |
| 23 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 24 | LCDR1 | RSSTGAVTTSNYAN |
| 25 | LCDR2 | GTNKRAP |
| 26 | LCDR3 | ALWYSNLWV |
| 27 | VL | QAVVTQEPSLTVSPGGTVTLTCRSSTGAVTTSNYANWVQQKPGQAPR GLIGGTNKRAPGVPARFSGSLLGDKAALTLLGAQPEDEAEYYCALW YSNLWVFGQGTKLTVL |
| 28 | LC | GQPKAAPSVTLFPPSSEELQANKATLVCLISDFYPGAVTVAWKADSSP VKAGVETTTPSKQSNNKYAASSYLSLTPEQWKSHRSYSCQVTHEGST VEKTVAPTECS |

| SEQ ID NO | | Knob (CH1 + CH2 + CH3) |
|---|---|---|
| 33 | | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLWCLVKGFYPSDIAVEWESNGQPENNYKTTPPVLDS DGSFFLYSKLTVDKSRWQQGNVFSCSVMHEALHNHYTQKSLSLSPG |
| | | K |

| SEQ ID NO | | Hole (CH1 + CH2 + CH3) |
|---|---|---|
| 34 | | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SRDELTKNQVSLSCAVKGFYPSDIAVEWESNGQPENNYKTTPPVLDS DGSFFLVSKLTVDKSRWQQGNVFSCSVMHEALHNHYTQKSLSLSPG K |

| SEQ ID NO | | sp34 antibody |
|---|---|---|
| 35 | VH | EVQLVESGGGLVQPKGSLKLSCAASGFTFNTYAMNWVRQAPGKGLE WVARIRSKYNNYATYYADSVKDRFTISRDDSQSILYLQMNNLKTEDT AMYYCVRHGNFGNSYVSWFAYWGQGTLVTVSS |
| 23 | HC | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPEAAGGPSVFLFPPKPKDTLMISRTPEVTC VVVDVSHEDPEVKFNWYVDGVEVHNAKTKPREEQYNSTYRVVSVL TVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYSKLTVDKSRWQQGNVFSCSVMHEALHNHYTQKSLSLSPG K |
| 36 | VL | QAVVTQESALTTSPGETVTLTCRSSTGAVTTSNYANWVQEKPDHLFT GLIGGTNKRAPGVPARFSGSLIGDKAALTITGAQTEDEAIYFCALWYS NLWVFGGGTKLTVL |
| 28 | LC | GQPKAAPSVTLFPPSSEELQANKATLVCLISDFYPGAVTVAWKADSSP VKAGVETTTPSKQSNNKYAASSYLSLTPEQWKSHRSYSCQVTHEGST VEKTVAPTECS |

| SEQ ID NO | | CLDN18.2 moiety HB37A6 in bispecific antibody |
|---|---|---|
| 37 | Heavy chain | EVQLLDSGGGLVQPGGSLRLSCAASGFTFSSYVMSWVRQAPGKGLN WVSTISHSGGSTYYADSVKGRFTISRDNSKNTLYLQMNSLRAEDTAV YYCAIDAPYYDILTGYRYWGQGTLVTVSSASTKGPSVFPLAPSSKSTS GGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPA PEAAGGPSVFLFPPKPKDTLMISRTPEVTCVVVDVSHEDPEVKFNWY VDGVEVHNAKTKPREEQYNSTYRVVSVLTVLHQDWLNGKEYKCKV SNKALPAPIEKTISKAKGQPREPQVYTLPPSRDELTKNQVSLWCLVKG FYPSDIAVEWESNGQPENNYKTTPPVLDSDGSFFLYSKLTVDKSRWQ |
| | | QGNVFSCSVMHEALHNHYTQKSLSLSPGK |
| 38 | Light chain | DIQMTQSPSTLSASVGDRVTITCRASQSISSWLAWYQQKPGKAPKLLI YKASSLESGVPSRFSGSGSGTEFTLTISSLQPDDFATYYCQQYNSYSYT FGQGTKLEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGEC |

| SEQ ID NO | | CD3 moiety HzSP34.24 in bispecific antibody |
|---|---|---|
| 39 | Heavy chain | EVQLVESGGGLVQPGGSLKLSCAASGFTFNTYAMNWVRQASGKGLE WVGRIRSKYNNYATYYADSVKDRFTISRDDSKSTLYLQMNSLKTEDT AVYYCARHGNFGQSYVSWFAYWGQGTTVTVSSASTKGPSVFPLAPS SKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTC PPCPAPEAAGGPSVFLFPPKPKDTLMISRTPEVTCVVVDVSHEDPEVK FNWYVDGVEVHNAKTKPREEQYNSTYRVVSVLTVLHQDWLNGKEY KCKVSNKALPAPIEKTISKAKGQPREPQVYTLPPSRDELTKNQVSLSC AVKGFYPSDIAVEWESNGQPENNYKTTPPVLDSDGSFFLVSKLTVDKS RWQQGNVFSCSVMHEALHNHYTQKSLSLSPGK |
| 40 | Light chain | QAVVTQEPSLTVSPGGTVTLTCRSSTGAVTTSNYANWVQQKPGQAPR GLIGGTNKRAPGVPARFSGSLLGDKAALTLLGAQPEDEAEYYCALW YSNLWVFGQGTKLTVLGQPKAAPSVTLFPPSSEELQANKATLVCLISD FYPGAVTVAWKADSSPVKAGVETTTPSKQSNNKYAASSYLSLTPEQW KSHRSYSCQVTHEGSTVEKTVAPTECS |

| SEQ ID NO | | CD3 moiety HzSP34.87 in bispecific antibody |
|---|---|---|
| 41 | Heavy chain | EVQLVESGGGLVQPGGSLKLSCAASGFTFNTYAMNWVRQASGKGLE WVGRIRSKYNNYATYYADSVKDRFTISRDDSKSTLYLQMNSLKTEDT AVYYCARHYNFGQSYVSWFAYWGQGTTVTVSSASTKGPSVFPLAPS SKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTC PPCPAPEAAGGPSVFLFPPKPKDTLMISRTPEVTCVVVDVSHEDPEVK FNWYVDGVEVHNAKTKPREEQYNSTYRVVSVLTVLHQDWLNGKEY KCKVSNKALPAPIEKTISKAKGQPREPQVYTLPPSRDELTKNQVSLSC AVKGFYPSDIAVEWESNGQPENNYKTTPPVLDSDGSFFLVSKLTVDKS RWQQGNVFSCSVMHEALHNHYTQKSLSLSPGK |
| 40 | Light chain | QAVVTQEPSLTVSPGGTVTLTCRSSTGAVTTSNYANWVQQKPGQAPR GLIGGTNKRAPGVPARFSGSLLGDKAALTLLGAQPEDEAEYYCALW YSNLWVFGQGTKLTVLGQPKAAPSVTLFPPSSEELQANKATLVCLISD FYPGAVTVAWKADSSPVKAGVETTTPSKQSNNKYAASSYLSLTPEQW KSHRSYSCQVTHEGSTVEKTVAPTECS |

| SEQ ID NO | | CD3 moiety HzSP34.97 in bispecific antibody |
|---|---|---|
| 42 | Heavy chain | EVQLVESGGGLVQPGGSLKLSCAASGFTFNTYAMNWVRQASGKGLE WVGRIRSKAGGYATYYADSVKDRFTISRDDSKSTLYLQMNSLKTEDT AVYYCARHGNFGQSYVSWFAYWGQGTTVTVSSASTKGPSVFPLAPS SKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTC PPCPAPEAAGGPSVFLFPPKPKDTLMISRTPEVTCVVVDVSHEDPEVK FNWYVDGVEVHNAKTKPREEQYNSTYRVVSVLTVLHQDWLNGKEY KCKVSNKALPAPIEKTISKAKGQPREPQVYTLPPSRDELTKNQVSLSC AVKGFYPSDIAVEWESNGQPENNYKTTPPVLDSDGSFFLVSKLTVDKS RWQQGNVFSCSVMHEALHNHYTQKSLSLSPGK |
| 40 | Light chain | QAVVTQEPSLTVSPGGTVTLTCRSSTGAVTTSNYANWVQQKPGQAPR GLIGGTNKRAPGVPARFSGSLLGDKAALTLLGAQPEDEAEYYCALW YSNLWVFGQGTKLTVLGQPKAAPSVTLFPPSSEELQANKATLVCLISD FYPGAVTVAWKADSSPVKAGVETTTPSKQSNNKYAASSYLSLTPEQW KSHRSYSCQVTHEGSTVEKTVAPTECS |

## Claims

1. A liquid antibody formulation, comprising:
(i) an anti-CLDN18.2/CD3 protein,
(ii) a buffer,
(iii) a stabilizer, and
(iv) a surfactant,
wherein the anti-CLDN18.2/CD3 bispecific antibody protein comprises a first antigen-binding domain and a second antigen-binding domain, wherein the first antigen-binding domain specifically binds to CLDN18.2, and the second antigen-binding domain specifically binds to CD3, wherein the first antigen-binding domain comprises three complementarity determining regions A1-HCDR1, A1-HCDR2, and A1-HCDR3 contained in an A1-VH set forth in SEQ ID NO: 4, and three complementarity determining regions A1-LCDR1, A1-LCDR2, and A1-LCDR3 contained in a VL set forth in SEQ ID NO: 9; the second antigen-binding domain comprises three complementarity determining regions A2-HCDR1, A2-HCDR2, and A2-HCDR3 contained in an A2-VH set forth in SEQ ID NO: 30, 22, or 32, and three complementarity determining regions A2-LCDR1, A2-LCDR2, and A2-LCDR3 contained in an A2-VL set forth in SEQ ID NO: 27;
preferably, the liquid antibody formulation has a pH of about 5.0-6.0, e.g., a pH of 5.2±0.2 or 5.5±0.2, preferably a pH of 5.2.

2. The liquid antibody formulation according to claim 1, wherein the concentration of the anti-CLDN18.2/CD3 protein in the liquid antibody formulation is about 0.1-100 mg/mL, preferably about 0.1-10 mg/mL, e.g., about 0.5 mg/mL, 1 mg/mL, 2 mg/mL, 3 mg/mL, 4 mg/mL, or 5 mg/mL.

3. The liquid antibody formulation according to claim 1 or 2, wherein the liquid antibody formulation comprises a buffer selected from a histidine-histidine hydrochloride buffer system, a citric acid-sodium citrate buffer system, an acetic acid-sodium acetate buffer system, and a phosphate buffer system; preferably, the buffer in the liquid antibody formulation is selected from histidine, histidine hydrochloride, and a combination thereof; preferably, the concentration of the buffer is about 0.01-10 mg/mL, preferably about 0.1-1 mg/mL, e.g., about 0.1 mg/mL, 0.2 mg/mL, 0.3 mg/mL, 0.4 mg/mL, 0.5 mg/mL, or 1 mg/mL.

4. The liquid antibody formulation according to any one of claims 1-2, wherein the stabilizer is selected from polyols (e.g., sorbitol, mannitol, and a combination thereof), saccharides (e.g., sucrose, trehalose, maltose, and a combination thereof), amino acids (e.g., arginine, arginine hydrochloride, methionine, glycine, proline, and a combination thereof), and any combination thereof,
for example, the stabilizer comprises one or more selected from:
- a polyol selected from sorbitol, mannitol, or a combination thereof;
- a saccharide selected from sucrose, trehalose, maltose, or a combination thereof; and
- an amino acid selected from arginine hydrochloride, methionine, glycine, proline, and a combination thereof.

5. The liquid antibody formulation according to any one of claims 1-4, wherein the stabilizer is selected from:
(i) a combination of about 20-40 mg/mL sorbitol and about 50-100 mM arginine, or
(ii) a combination of about 30-60 mg/mL sucrose and about 50-100 mM arginine, or
(iii) about 30-70 mg/mL, preferably about 50 mg/mL, sorbitol.

6. The liquid antibody formulation according to any one of claims 1-4, wherein the surfactant in the liquid antibody formulation is selected from a polysorbate surfactant, poloxamer, polyethylene glycol, or a combination thereof, preferably polysorbate-80.

7. The liquid antibody formulation according to any one of claims 1-5, wherein the concentration of the surfactant is about 0.01-5 mg/mL, preferably about 0.1-1 mg/mL, e.g., about 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL, more preferably about 0.2 mg/mL.

8. The liquid antibody formulation according to any one of claim 7, further comprising a chelating agent, wherein the chelating agent is selected from edetate disodium, nitrilotriacetic acid, diethylenetriaminepentaacetic acid, citric acid, tartaric acid, gluconic acid, hydroxyethylethylenediaminetriacetic acid, and dihydroxyethylglycine; preferably, the chelating agent is selected from edetate disodium;
preferably, the chelating agent is at about 0.008-0.018 mg/mL, e.g., about 0.008 mg/mL, 0.009 mg/mL, 0.010 mg/mL, 0.012 mg/mL, 0.014 mg/mL, or 0.018 mg/mL, preferably 0.01 mg/mL.

9. The liquid antibody formulation according to any one of claims 1-8, wherein in the anti-CLDN18.2/CD3 protein,
the first antigen-binding domain comprises A1-HCDR1, A1-HCDR2, and A1-HCDR3 set forth in the amino acid sequences of SEQ ID NOs: 1, 2, and 3, respectively, and A1-LCDR1, A1-LCDR2, and A1-LCDR3 set forth in the amino acid sequences of SEQ ID NOs: 6, 7, and 8, respectively; and
the second antigen-binding domain comprises:
(i) A2-HCDR1, A2-HCDR2, and A2-HCDR3 set forth in the amino acid sequences of SEQ ID NOs: 19, 20, and 29, respectively, and LCDR1, LCDR2, and LCDR3 set forth in the amino acid sequences of SEQ ID NOs: 24, 25, and 26, respectively; or
(ii) A2-HCDR1, A2-HCDR2, and A2-HCDR3 set forth in the amino acid sequences of SEQ ID NOs: 19, 20, and 21, respectively, and LCDR1, LCDR2, and LCDR3 set forth in the amino acid sequences of SEQ ID NOs: 24, 25, and 26, respectively; or
(iii) A2-HCDR1, A2-HCDR2, and A2-HCDR3 set forth in the amino acid sequences of SEQ ID NOs: 19, 31, and 21, respectively, and LCDR1, LCDR2, and LCDR3 set forth in the amino acid sequences of SEQ ID NOs: 24, 25, and 26, respectively.

10. The liquid antibody formulation according to any one of claims 1-9, wherein the first antigen-binding domain comprises a heavy chain variable region and/or a light chain variable region, wherein the heavy chain variable region
(i) comprises or consists of an amino acid sequence having at least 90%, 91%, 92%, 93%, 94%, 95%, 96%, 97%, 98%, or 99% identity to the amino acid sequence selected from SEQ ID NO: 4; or
(ii) comprises or consists of the amino acid sequence selected from SEQ ID NO: 4; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 4, wherein preferably, the amino acid changes do not occur in the CDRs; and/or
the light chain variable region
(i) comprises or consists of an amino acid sequence having at least 90%, 91%, 92%, 93%, 94%, 95%, 96%, 97%, 98%, or 99% identity to the amino acid sequence selected from SEQ ID NO: 9; or
(ii) comprises or consists of the amino acid sequence selected from SEQ ID NO: 9; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 9, wherein preferably, the amino acid changes do not occur in the CDRs.

11. The liquid antibody formulation according to any one of claims 1-8, wherein the anti-CLDN18.2/CD3 is an IgG antibody (e.g., an IgG4 subtype antibody), and preferably comprises a heavy chain sequence of SEQ ID NO: 9 or a sequence having at least 90%, 95%, 98%, or 99% identity thereto, and a light chain sequence of SEQ ID NO: 10 or a sequence having at least 90%, 95%, 98%, or 99% identity thereto.

12. The liquid antibody formulation according to any one of claims 1-11, wherein the second antigen-binding domain comprises a heavy chain variable region and/or a light chain variable region, wherein the heavy chain variable region
(i) comprises or consists of an amino acid sequence having at least 90%, 91%, 92%, 93%, 94%, 95%, 96%, 97%, 98%, or 99% identity to the amino acid sequence selected from SEQ ID NO: 30, 22, or 32; or
(ii) comprises or consists of the amino acid sequence selected from SEQ ID NO: 30, 22, or 32; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 30, 22, or 32, wherein preferably, the amino acid changes do not occur in the CDRs;
and/or
the light chain variable region
(i) comprises or consists of an amino acid sequence having at least 90%, 91%, 92%, 93%, 94%, 95%, 96%, 97%, 98%, or 99% identity to the amino acid sequence selected from SEQ ID NO: 27; or
(ii) comprises or consists of the amino acid sequence selected from SEQ ID NO: 27; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence selected from SEQ ID NO: 27, wherein preferably, the amino acid changes do not occur in the CDRs.

13. The liquid antibody formulation according to any one of claims 1-11, further comprising a heavy chain constant region and/or a light chain constant region.

14. The liquid antibody formulation according to any one of claims 1-12, being a bispecific antibody with an IgG-like structure.

15. The liquid antibody formulation according to any one of claims 1-13, wherein the heavy chain constant region of the antibody is from IgG1, IgG2, IgG3, or IgG4, preferably IgG1.

16. The liquid antibody formulation according to any one of claims 1-14, wherein the anti-CLDN18.2/CD3 is recombinantly expressed in 293 cells or CHO cells.

17. The liquid antibody formulation according to any one of claims 1-16, wherein the liquid antibody formulation comprises:
(i) about 0.1-100 mg/mL anti-CLDN18.2/CD3 protein;
(ii) about 0.1-2 mg/mL histidine;
(iii) about 30-70 mg/mL sorbitol;
(iv) about 0.1-1 mg/mL polysorbate 80; and
(v) optionally, about 0.008-0.018 mg/mL edetate disodium,
wherein the liquid formulation has a pH of about 5.0-5.5, e.g., about 5.2;
for example, the liquid antibody formulation comprises:
(i) about 0.1-10 mg/mL, e.g., 0.1-1 mg/mL, anti-CLDN18.2/CD3 protein;
(ii) about 1.55 mg/mL histidine;
(iii) about 40-60 mg/mL sorbitol or sucrose, preferably 50 mg/mL sorbitol or 30-50 mg/mL sucrose;
(iv) about 0.2-0.8 mg/mL, e.g., 0.2-0.6 mg/mL, polysorbate 80; and
(v) about 0.008-0.018 mg/mL, e.g., 0.01 mg/mL, edetate disodium,
wherein the liquid formulation has a pH of about 5.0-5.5, e.g., about 5.2;
or the liquid antibody formulation comprises:
(i) about 1 mg/mL anti-CLDN18.2/CD3 protein, about 1.55 mg/mL histidine, about 50.00 mg/mL sorbitol, and about 0.2 mg/mL polysorbate 80, with a pH of about 5.2; or
(ii) about 50 mg/mL anti-CLDN18.2/CD3 protein, about 1.55 mg/mL histidine, about 50.00 mg/mL sorbitol, about 0.01 mg/mL edetate disodium, and about 0.2 mg/mL polysorbate 80, with a pH of about 5.2; or
(iii) about 50 mg/mL anti-CLDN18.2/CD3 protein, about 0.82 mg/mL sodium acetate, about 50.00 mg/mL sorbitol, and about 0.2 mg/mL polysorbate 80, with a pH of 5.2.

18. The liquid antibody formulation according to any one of claims 1-13, wherein the formulation is stable after storage, e.g., at 25 °C for at least 2 months, or at 40±2 °C for 1 month, and preferably has one or more of the following characteristics:
(i) a main peak change less than 1%, and/or a purity greater than 96%, preferably greater than 97% or 98% in the formulation as measured by SEC-HPLC;
(ii) a main peak change less than 2%, and/or a purity greater than 96%, preferably greater than 97% or 98% in the formulation as measured by non-reduced CE-SDS;
(iii) a sum of changes in species (main species, acidic species, and basic species) not more than 40% and/or a change in the main species not more than 20% of the anti-CLDN18.2/CD3 protein in the formulation relative to an initial value on day 0 of storage as measured by CEX-HPLC, for example, a sum of changes not more than about 40% (e.g., not more than 30%) and/or a change in the main species not more than about 20% (e.g., not more than 15%) after storage at 40±2 °C for 1 month, or a sum of changes not more than about 20% (e.g., about 15%) or a change in the main species not more than about 15% (e.g., not more than about 10%) after storage at 25 °C for 2 months; and
(iv) a relative binding activity of the anti-CLDN18.2/CD3 protein in the formulation of 70%-130%, e.g., 90%-110%, relative to an initial value on day 0 of storage as measured by ELISA;
more preferably, the formulation is stable at shaking and/or repeated freezing-thawing.

19. The liquid antibody formulation according to any one of claims 1-15, wherein the liquid formulation is an injection, preferably for use in subcutaneous injection or intravenous injection, or an infusion, e.g., for use in intravenous infusion.

20. A solid antibody formulation obtained by solidifying the liquid antibody formulation according to any one of claims 1-19, wherein the solid antibody formulation is, e.g., in the form of a lyophilized powder for injection.

21. A delivery device, comprising the liquid antibody formulation according to any one of claims 1-19 or the solid antibody formulation according to claim 15.

22. A pre-filled syringe, comprising the liquid antibody formulation according to any one of claims 1-19 or the solid antibody formulation according to claim 15 for use in intravenous or intramuscular injection.

23. Use of the liquid antibody formulation according to any one of claims 1-20 or the solid antibody formulation according to claim 15 in preparing a delivery device, a pre-filled syringe, or a medicament for treating or preventing a tumor or a pathogen infection in a subject.
